# EUROPÄISCHE PATENTANMELDUNG

(11) **EP 4 036 206 A1**
(43) Veröffentlichungstag der Anmeldung: **03.08.2022**
(21) Anmeldenummer: 21153820.2
(22) Anmeldetag: 27.01.2021
(51) Int. Cl.: C12M 1/00

(54) **INKUBATOR, SYSTEM UND VERFAHREN**

(71) Anmelder: Eppendorf AG, 22339 Hamburg (DE)
(72) Erfinder: JOLIE, Christoph, 22339 Hamburg (DE); PAULSEN, Benjamin, 22339 Hamburg (DE); HELLWEG, Wolf Lukas, 22339 Hamburg (DE)
(74) Vertreter: Kirchner, Christian

(57) **Zusammenfassung**

Die Erfindung bezieht sich auf einen Inkubator für lebende Zellkulturen, ein Verfahren zur Arbeit mit einem Inkubator und ein System mit einem Inkubator, bei dem ein Objektverfolgungssystem realisiert ist.

## Beschreibung

Die Erfindung bezieht sich auf einen Inkubator für lebende Zellkulturen, ein Verfahren zur Arbeit mit einem Inkubator und ein System mit einem Inkubator.

Die Erfindung betrifft einen Inkubator für das überwachte Wachstum von biologischen Zellen. Die Erfindung betrifft zudem ein System und ein Verfahren für das überwachte Wachstum von biologischen Zellen.

Mit solchen Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen CO₂-Inkubatoren. Die Atmosphäre wird durch Luft mit einem bestimmten CO₂- und O₂-Gehalt und einer bestimmten Luftfeuchtigkeit gebildet, eine geeignete Temperatur ist oftmals 37 °C.

Das Wachstum der Zellen hängt insbesondere kritisch von der Konstanz der atmosphärischen Bedingungen im Inkubator ab. Störungen der Inkubatoratmosphäre können sich negativ auf das Wachstum der Zellen auswirken. In einem "ideal" ausgerüsteten Labor würde man jedem einzelnen Nutzer für jede zu inkubierende Probe eine separat zugängliche Inkubationskammer zur Verfügung stellen. Dies ist aber aus Gründen der Kosteneffizienz nicht realistisch. In der Praxis der Labore ist es üblich, dass ein einzelner Inkubator (oder wenige Inkubatoren) mit einer einzigen Inkubationskammer und einem oder mehrerer Lagerbereiche auf einer oder mehreren Lagerplatten in der Inkubationskammer für die Verwendung durch mehrere Benutzer vorgesehen ist. Die Häufigkeit des Öffnens der Kammertüre des Inkubators und damit des Eingriffs in die geregelte Atmosphäre des Inkubators skaliert mit der Anzahl der Nutzer und zudem mit der Anzahl der dort inkubierten Proben. Die Intensität der Störung hängt zudem von der Dauer der Türöffnung ab. Je mehr Zeit ein Nutzer für den Zugriff auf den Innenraum der Inkubatorkammer benötigt, desto länger bleibt die Tür geöffnet. Es gibt verschiedene Nutzungsszenarien des Inkubators, die durch Komplikationen eine erhöhte Zugriffszeit erfordern können:

### Szenario A) Einstellen neuer Objekte in den Inkubator

Stellt ein Nutzer einen oder mehrere Objekte, insbesondere Zellkulturbehälter, erstmals in den Inkubator ein, benötigt er einen freien Lagerplatz in einem Lagerbereich. Sind wegen ungeordneter Lagerung keine freien Lagerplätze zugänglich, benötigt der Nutzer Zeit um diesen Lagerplatz zu schaffen; je sorgfältiger er dabei im Inkubator bereits vorhandene Objekte (Bestandsobjekte) verschiebt bzw. umräumt, dies eventuell sogar schriftlich dokumentiert, desto zeitaufwändiger wird der Vorgang. Erweist es sich dabei, dass in der Inkubatorkammer kein ausreichender Lagerplatz mehr verfügbar ist, so wiederholt der Nutzer sein Vorgehen bei einem eventuell vorhandenen weiteren Kompartiment der Inkubatorkammer oder bei einem Ersatzinkubator des Labors. Dadurch verlängert sich der Zeitraum des Zustands einer geöffneten Inkubatortüre, also die Dauer der Exposition des Kammerinneren zur Umgebung (Expositionsdauer).

### Szenario B) Prüfen von Zellkulturen

Prüft ein Nutzer die von ihm zuvor in den Inkubator eingestellten Zellkulturen, z.B. um die Qualität des Zellmediums oder den Zustand des Wachstums zu beurteilen, so wird der Nutzer zunächst den betreffenden Zellkulturbehälter im Inkubator suchen. Möglicherweise kann er sich nicht an den von ihm gewählten Lagerplatz erinnern oder andere Nutzer haben diesen Lagerplatz verändert - beides verlängert die Expositionsdauer, d.h. die Öffnungsdauer des Inkubators und die damit eingehenden Atmosphärenverluste. Je sorgfältiger der Nutzer dabei Bestandsobjekte verschiebt bzw. umräumt, desto zeitaufwändiger wird der Suchvorgang.

### Szenario C) Entnehmen der Objekte aus dem Inkubator

Auch in diesem Fall muss der Nutzer das entsprechende Objekt erst suchen. Es gelten die in b) genannten zeitverzögernden Faktoren.

Je häufiger zudem ein Inkubator geöffnet wird, desto höher ist das Risiko der Kontamination des Innenraums. Es gibt auch Fälle, beispielsweise in der Forensik oder der Reproduktionsmedizin, in denen der Wert einer einzelnen Probe, insbesondere der in einem Zellkulturgefäß befindlichen Zelle/n, viel höher einzuschätzen ist als, beispielsweise, der Wert des gesamten Inkubators, so dass ein kontaminationsbedingter Verlust der Probe unbedingt zu vermeiden ist. In jedem Fall erhöht die Kontaminationshäufigkeit das Risiko von Arbeitsausfall, erhöhten Kosten und eines zusätzlichen Wartungsaufwands. Nach einer Kontamination eines Inkubators muss die Kammer gereinigt und sterilisiert werden, bevor der Inkubator weiter genutzt werden kann. In dieser Zeit ist, insofern kein Ersatzinkubator verfügbar ist, ist die Arbeit mit Zellkulturen unterbrochen.

In Laboren besteht daher grundsätzlich das Bedürfnis, den Zeitraum möglichst kurz zu halten, während dem die Inkubatortüre geöffnet ist, und zudem, die Häufigkeit des Öffnens der Inkubatorkammer möglichst gering zu halten.

Die der vorliegenden Erfindung zugrunde liegende Aufgabe ist es, einen effizient nutzbaren Inkubator bereitzustellen, der insbesondere es ermöglicht die Expositionsdauer, also den Zeitraum des Zustands einer geöffneten Inkubatortüre, und damit die Dauer der Exposition des Inneren der Inkubatorkammer zur Umgebung des Inkubators gering zu halten.

Die Erfindung löst diese Aufgabe durch den Inkubator gemäß Anspruch 1, das System gemäß Anspruch 12, und das Verfahren gemäß Anspruch 14. Bevorzugte Ausgestaltungen sind insbesondere Gegenstände der Unteransprüche.

Der erfindungsgemäße Inkubator zur Inkubation lebender Zellkulturen, weist auf:
eine Inkubatorkammer zur Aufnahme von Objekten, insbesondere Zellkulturbehältern, die eine verschließbare Kammeröffnung zur Auf- und Entnahme der Objekte und mindestens einen Lagerbereich zur Lagerung der Objekte aufweist,
ein bildbasiertes Objektverfolgungssystem zur Verfolgung von Positionsänderungen mindestens eines in die Inkubatorkammer eingebrachten Objektes ausgehend von dessen Startposition in einem Startbild eines Lagerbereichs bis zu dessen Endposition in einem Endbild des Lagerbereichs mittels Videodaten, wobei das Objektverfolgungssystem
mindestens eine Datenverarbeitungseinrichtung, einen Datenspeicher und mindestens eine Kamera aufweist, die dazu eingerichtet ist, einen Innenraum der Inkubatorkammer zu überwachen, insbesondere einen Lagerbereich, sowie das Startbild, das Endbild und die Videodaten bereitzustellen,
wobei die Datenverarbeitungseinrichtung dazu programmiert ist,
   - dem in den Innenraum eingebrachten mindestens einen Objekt Identifikationsdaten zuzuordnen, (dieser Schritt wird auch als Objektregistrierung bezeichnet)
   - die Startposition des mindestens einen Objektes aus dem Startbild des Lagerbereichs zu ermitteln,
   - die Positionsänderungen des mindestens einen Objektes zwischen der Startposition und einer Endposition durch Auswertung der Videodaten zu ermitteln,
   - die Endposition des mindestens einen Objektes im Endbild des Lagerbereichs zu ermitteln, und
   - die Endposition des mindestens einen Objektes im Lagerbereich in Abhängigkeit von den Identifikationsdaten als ID-Positionsdaten in dem Datenspeicher zu speichern.

Der erfindungsgemäße Inkubator "kennt" das beim Einsetzen in den Inkubator "registrierte" Objekt anhand eines internen Systemnamens, nämlich der diesem Objekt zugeordneten Identifikationsdaten (kann eine Identifikationsnummer sein, oder ein anderer Identifikationscode aus beliebigen Zeichen bzw. Informationen). Der Inkubator kennt aufgrund der Positionsverfolgung die Endposition des Objektes nach jeder Positionsänderung, die beispielsweise erzeugt wird, wenn ein anderer Benutzer zu einem späteren Zeitpunkt das Objekt im Inkubator verschiebt. Der Inkubator "merkt sich" die Endposition des Objektes, und vorzugsweise weiterer Objekte bzw. jedes weiteren Objektes in der Inkubatorkammer. Das Ziel der Objektverfolgung ist es, dem an der Startposition lokalisierten Objekt nach dessen Positionsänderungen, d.h. dessen Bewegung im Kamerasichtbereich, eindeutig dessen Endposition zuordnen zu können, an dem die Positionsänderungen Null sind und die Bewegung des Objekts beendet ist. Nicht primär relevant ist es, den gesamten Bewegungspfad des durch die Positionsänderungen bewegten Objektes präzise beschreiben zu können. Dies kann jedoch in einer bevorzugten Ausgestaltung der Erfindung vorgesehen sein.

Bildverarbeitende Objektverfolgungstechniken sind grundsätzlich bekannt, zum Beispiel beim Einsatz in Drohnen oder Fahrassistenzsystemen zur Fahrzeug- oder Personenverfolgung. Die Objektverfolgung (englisch: "object tracking") basiert auf der Bildverarbeitung bzw. Bildauswertung von Videobilddaten. Solche Verfahren der Objektverfolgung lassen sich mit relativ einfachen Mitteln wie geeigneten Kameras und Bildverarbeitungsalgorithmen umsetzten. Die theoretischen Grundlagen und deren Nutzung zur praktischen Realisierung von Objektverfolgungstechniken sind bekannt, insbesondere die Realisierung von "single object tracking" und "multiple object tracking" (z.B.: "Fundamentals of Object Tracking", S. Challa et al., Cambridge University Press, 2011). Auch sofort verwendbare Bildverarbeitungsalgorithmen zur Objektverfolgung sind frei verfügbar (OpenCV.org) und gut dokumentiert. OpenCV (englische Abk. für Open Computer Vision) ist eine freie Programmbibliothek (BSD Lizenz) mit Algorithmen für die Bildverarbeitung und Computer Vision. Die Programmbibliothek OpenCV beinhaltet auch Funktionen zur Verfolgung mehrerer Objekte in Echtzeit. Die Anwendung von Objektverfolgung in Inkubatoren wurde bislang nicht publiziert und stellt eine Innovation dar.

Eine typische Arbeitsweise von bildverarbeitender Objektverfolgung, die vorzugsweise auch im Objektverfolgungssystem gemäß vorliegender Erfindung zum Einsatz kommt, basiert auf der Auswertung der zeitlichen Abfolge von Bildern. Ein typischer Programmcode zur Objektverfolgung verwendet eine "Bounding Box" als Ausgabeformat, um ein Objekt in einem Bild zu identifizieren, dessen Kollisionsgrenzen festzulegen und insbesondere zu lokalisieren. In der digitalen Bildverarbeitung bezeichnet die "Bounding Box" die Koordinaten des rechteckigen Rahmens, der ein im digitalen Bild gezeigtes Objekt größtenteils oder vollständig umschließt. Durch die Verwendung von Bounding Boxes bei der Objektverfolgung wird diese effizienter, da die Bildauswertung mittels eines solchen numerischen Hilfsmittels, insbesondere im Vergleich mit Algorithmen der Umrisserkennung von Objekten weniger Rechenschritte und damit eine geringere Rechenleistung erfordert. Zudem lassen sich die entsprechenden Algorithmen mithilfe von spezialisierten Grafikprozessoren (GPUs) effizient und kostengünstig ausführen. Geeignete Programmierschnittstellen (APIs) zur Objektverfolgung mittels Bounding Boxes stehen in der Programmbibliothek OpenCV unter den Bezeichnungen BOOSTING, CSRT, GOTURN, KCF, MEDIANFLOW, MOSSE, MIL, TLD zur Verfügung. Entsprechend stehen in OpenCV Programmbibliotheken ("MultiTracker") für das zeitgleiche Verfolgen von mehreren Objekten ("multiple object tracking") zur Verfügung. Als Alternative dazu sind Deep Learning Algorithmen zum Multi-Object Tracking (MOT) nach dem "tracking-by-detection" Prinzip bekannt.

Es ist aber auch möglich und bevorzugt, dass zur Objektverfolgung eine Ermittlung der Kontur des zu verfolgenden Objektes im Bild durchgeführt wird, und insbesondere der Trennung von Objekt (Vordergrund) und Hintergrund durch Hintergrundsubtraktion.

Das Leistungspotential eines Objektverfolgungssystems beruht einerseits auf einem zuverlässigen automatischen Identifizieren eines Objekts im Inkubator in verschiedenen typischen Nutzungsszenarien eines Inkubators, die nachfolgend beschrieben werden. Andererseits ist der Ansatz effizient, da auf Seiten des Objekts keine besonderen Anpassungen erforderlich sind. Insbesondere muss das Objekt keine passiven (Code, Beschriftung) oder aktiven (z.B. einen Sender) Identifikationshilfsmittel beinhalten. Es lassen sich vielmehr die üblichen Objekte (Zellkulturbehälter, Geräte etc.) mit dem Inkubator verwenden, insbesondere unabhängig von Hersteller und äußerlicher Erscheinung. Insbesondere ist der erfindungsgemäße Inkubator in der Lage, Objekte mit vollkommen identischem Aussehen durch die Verfolgung zu unterscheiden.

Mögliche Szenarien bei der Änderung der Belegung in einem Inkubator sind:
I. Einstellen neuer Objekte
II. Entnahme von Objekten
III. Tür wird geöffnet und es werden Objekte nur verschoben, ohne eines zu entnehmen oder neu einzustellen.

Subkonditionen:
i) Objekt/e werden verschoben
ii) Objekt/e werden nicht verschoben
iii) Mehrere Objekte werden eingestellt/entnommen (Reihenfolge).

Annahme: alle Zellkulturbehälter sehen äußerlich gleich aus. Die der Entwicklung der Erfindung zugrunde liegende Frage war insbesondere, welche bildbasierten Methoden in Frage kommen, insbesondere ob Standbilder ausreichen, um in üblichen Nutzungsszenarien eines Inkubators eine Objektidentifikation zu ermöglichen.

Es wird für das Szenario I. (neues Objekt soll in die Inkubatorkammer eingesetzt werden) zunächst angenommen, dass es vor der Öffnung der Inkubatortüre ein aktuelles, von einer im Inkubator platzierten Kamera aufgenommenes Standbild des Lagerbereichs in der Inkubatorkammer gibt, welches das neue Objekt noch nicht zeigt.

Wird das neue Objekt ohne Verschieben der Bestandsobjekte (bereits im Lagerbereich angeordnete und lokalisierte Objekte) in die Inkubatorkammer eingestellt (Fall I.ii)), lässt sich das neue Objekt (problemlos eindeutig) über das nächste Standbild (nach Schließen der Inkubatortüre) identifizieren. Eine Objektverfolgung ist für den Fall I.ii) nicht notwendig. Entsprechendes gilt für II.ii): im Fall der Entnahme eines Objektes ist dessen Identifizierung eindeutig möglich aus der Evaluierung der Standbilder vor und nach Türöffnung.

Wird das neue Objekt eingestellt und werden dabei Bestandsobjekte verschoben (Fall I.i)), lässt sich das neue Objekt nicht eindeutig über das nächste Standbild identifizieren. Standortinformationen über die bereits registrierten Bestandsobjekte gehen verloren. Dasselbe gilt für die Entnahme eines Objektes und dabei erfolgendes Verschieben der Bestandsobjekte (Fall II.i)). Für das Verschieben (Kondition i)) kommt das Konzept der Objektverfolgung zu tragen.

Werden mehrere Objekte (Fall iii)) unter der Bedingung i), d.h. ohne Verschieben der Bestandsobjekte, eingestellt, lassen sich zwar die neuen Objekte per vorher-nachher-Standbilder einfach identifizieren, es geht aber die Information über die Reihenfolge des Einstellens verloren. Will man diese Information erhalten, benötigt man die Objektverfolgung. Entsprechendes gilt für den Fall der Entnahme mehrerer Objekte im Fall i) + iii). Die Reihenfolge des Hereinstellens ist aber selten entscheidend. Insbesondere kommt es bei Inkubationsdauern von z.B. 48h nicht auf die Sekunde an. Insbesondere aber, wenn auch die Objektzuordnung für den Fall aufgelöst werden soll, dass Objekte mehrerer Personen bei geöffneter Inkubatortüre hintereinander in die Inkubatorkammer eingestellt werden oder wenn das Umräumen von Objekten aus einem Regalfach der Inkubatorkammer in eine andere Inkubatorkammer verfolgt werden soll, kann die Erfassung der Reihenfolge beziehungsweise die Erfassung des zeitlichen Verlaufs des Versetzens von Objekten gewünscht sein. Für ein "Basistracking", bei dem die Anwender bestimmte Regeln der Bedienung des Inkubators beachten, kann auf die Erfassung der Reihenfolge verzichtet werden.

Da im Betrieb eines Inkubators ein Verschieben von Bestandsobjekten eher die Regel als die Ausnahme ist, reicht in diesem Fall eine Auswertung der vorher-nachher-Standbilder des Lagerbereichs nicht aus.

Eine Frage bei der Entwicklung eines Objektverfolgungssystems in einem Inkubator ist insbesondere: Wann wird ein Objekt identifiziert, d.h. zu welchem Zeitpunkt bzw. bei welchem Ereignis werden dem Objekt eine Identifikationsdaten zugeordnet? In den meisten Anwendungsszenarien (abgesehen von einem Fall wie iii)+i), bei dem es ggf. doch auf die Erfassung der Reihenfolge des Einstellens von Objekten ankommt) reicht es aus, diese Identifikationsdaten zu erheben, wenn die neuen Objekte eingestellt wurden und dabei eventuell Bestandsobjekte verschoben wurden. Denn das Verschieben der bereits registrierten Bestandsobjekte erfolgt unter der Maßnahme der Objektverfolgung. Im nächsten Standbild, also insbesondere bei geschlossener Inkubatortüre, kann dann die Registrierung der neuen Objekte anhand des Standbildes erfolgen. Falls doch die Erfassung der Reihenfolge gewünscht ist, kann in dem Moment, in dem ein Objekt erstmals in den Sichtbereich der Kamera eintritt, also erstmals in einem von der Kamera aufgenommenen Bild (ein Startbild, das hier ein Videobild ist) auftritt, wird dieses registriert werden, also dem Objekt eine ID-Nummer zugeordnet, und es wird dieses Objekt verfolgt bis zu seiner Endposition, das eventuelle Verschieben von Bestandsobjekten oder deren Entnahme wird dabei vorzugsweise auch verfolgt.

In einem anderen praxisrelevanten Szenario wird von einer Belegung des Lagerbereichs (oder mehrerer Lagerbereiche) mit einem oder mehreren Objekten (Bestandsobjekten) ausgegangen, die anhand eines ersten Startbildes (in diesem Fall z.B. ein Standbild) registriert werden. Es muss hier nur die eventuell stattfindende Verschiebung dieser Bestandsobjekte verfolgt werden. Die Bewegung neu einzustellender Objekte muss beim Vorgang des Einstellens hier nicht verfolgt werden, denn deren Registrierung kann wieder im nächsten Standbild erfolgen. Die entsprechende Präsenz dieser neuen Objekte in den Videodaten kann somit ignoriert werden. In diesem Szenario geht die Information über die Reihenfolge des Einstellens mehrerer Objekte während einer Türöffnung verloren, aber diese Information wird auch nicht zwingend benötigt.

Die Erfindung schlägt demnach vor, eine Objektverfolgung zu implementieren, um die korrekte Lokalisation von Objekten, je nach Bedarf, in verschiedenen oder allen Situationen zu gewährleisten.

Die Datenverarbeitungseinrichtung ist insbesondere dazu programmiert, dem in den Innenraum eingebrachten mindestens einen Objekt Identifikationsdaten zuzuordnen. Dies bedeutet insbesondere, dass in den Bilddaten (Standbilder oder Videodaten) der Kamera ein neues Objekt detektiert wird. Insbesondere wird ein neues Objekt dann detektiert, wenn dieses in den Kamerasichtbereich der Kamera von außen hineinbewegt wird. Mit dem Detektieren des Objektes können diesem einerseits Identifikationsdaten zugeordnet werden (Registrierung), andererseits auch Positionsdaten. Die Positionsdaten, insbesondere Start- und Endposition eines Objektes, werden insbesondere mit Bezug auf ein internes Koordinatensystem bestimmt, anhand dem auch die Lage des mindestens einen Lagerbereichs und damit auch die Lage des mindestens einen Objektes zu dem mindestens einen Lagerbereich definiert wird. Diese Lageinformationen sind insbesondere dafür von Bedeutung, wenn dem Benutzer auf einem Display die Position des mindestens einen Objektes in dem mindestens einen Lagerbereich bzw. In der Inkubatorkammer grafisch veranschaulicht werden soll.

Diese Identifikationsdaten können vorbestimmt sein, zufallsgeneriert sein, oder von einem Benutzer vorgegeben sein, insbesondere solange sie geeignet sind, das neu in die Inkubatorkammer eingestellte Objekt gegenüber den Identifikationsdaten der anderen Bestandsobjekte eindeutig zu unterscheiden. Die Identifikationsdaten können auch vorbestimmt sein und lediglich ausgewählt werden. Letzteren Fall umfasst der Begriff "zuordnen" ebenso wie die Neuerstellung der Identifikationsdaten.

Die Datenverarbeitungseinrichtung ist insbesondere dazu programmiert, die Startposition des mindestens einen Objektes aus dem Startbild des Lagerbereichs zu ermitteln. Das Startbild ist vorzugsweise ein Standbild, das in einem Standbildmodus der Kamera aufgenommen wird. Es kann auch ein aus Videodaten gewonnenes Einzelbild sein, insbesondere ein Videoframe. Die Datenverarbeitungseinrichtung ist insbesondere dazu programmiert, eine einhüllende oder markierende Linienfigur, vorzugsweise Rechteck, oder einen einhüllenden oder markierenden Körper, oder insbesondere eine Bounding Box des Objektes, oder eine Außenkontur des Objektes, in dem Startbild festzulegen und, insbesondere, das Objekt als den von der einhüllenden oder markierenden Linienfigur, insbesondere von der Bounding Box oder einer Außenkontur eingeschlossenen bzw. markierten Bildbereich zu definieren.

Die Datenverarbeitungseinrichtung ist insbesondere dazu programmiert, die Positionsänderungen des mindestens einen Objektes durch Auswertung der Videodaten zu ermitteln. Die Datenverarbeitungseinrichtung ist insbesondere dazu programmiert, die Bewegung des im Startbild mittels der Bounding Box definierten Objektes zu verfolgen. Die Datenverarbeitungseinrichtung ist insbesondere dazu programmiert, die Bewegung des im Startbild mittels der Bounding Box definierten Bildbereichs, der das Objekt enthält, zu erkennen, indem die Positionsänderungen dieses Bildbereichs von Frame zu Frame ermittelt werden. Dabei kann insbesondere eine Verfolgung der Bounding Box verwendet werden, um den sich aufgrund der Objektbewegung positionsverändernden Bildbereich zu ermitteln. Videodaten enthalten insbesondere Informationen, mittels denen sich die das Video kennzeichnenden Einzelbilder ("frames", die im Falle der Darstellung des Videos mit bestimmter Anzahl pro Zeit also "framerate" dargestellt werden) rekonstruieren lassen. Im Fall nicht komprimierter Videodaten können letztere auch die komplette Sequenz von Bilddaten enthalten, wobei ein "Satz" Bilddaten jeweils ein Einzelbild repräsentiert. Im Fall komprimierter Bilddaten werden eventuell auch/nur zeitliche Veränderungen von Pixeln des Kamerabilds erfasst.

Die Datenverarbeitungseinrichtung ist insbesondere dazu programmiert, aus dem Startbild die Startposition des Objektes im Lagerbereich zu ermitteln. Die Startposition kann insbesondere dadurch bestimmt sein, dass das in einem ersten Frame einer Bilderserie zuvor unbewegte Objekt im nachfolgenden Frame eine Positionsänderungen des Objekts zeigt. Der erste Frame, in dem das Objekt gegenüber den vorangegangenen Frames eine Positionsänderung aufweist, kann als das Startbild definiert werden. Da die Bewegung des Objektes zu einem Zeitpunkt T1 beginnt und zu einem Zeitpunkt T2 endet, kann ein Bild (Standbild oder Videobild, auch ein Bild gewonnen aus superpositionierten Bildern), das vor dem Zeitpunkt T1 erfasst wird, als Startbild herangezogen werden, aus dem die Startposition des mindestens einen Objektes festgelegt wird.

Die Datenverarbeitungseinrichtung ist insbesondere dazu programmiert, aus den Positionsänderungen die Endposition des mindestens einen Objektes im Endbild des Lagerbereichs zu ermitteln. Die Endposition kann insbesondere dadurch bestimmt sein, dass von Frame zu Frame keine Positionsänderungen des Objekts mehr bestimmt werden. Der erste Frame, in dem das Objekt gegenüber den vorangegangenen Frames keine Positionsänderung mehr aufweist, kann als das Endbild definiert werden. Da die Bewegung des Objektes zu einem Zeitpunkt T1 beginnt und zu einem Zeitpunkt T2 endet, kann ein Bild (Standbild oder Videobild, auch ein Bild gewonnen aus superpositionierten Bildern), das ab dem Zeitpunkt T2 erfasst wird, als Endbild herangezogen werden, aus dem die Endposition des mindestens einen Objektes festgelegt wird. Die Endposition kann insbesondere durch den Zeitpunkt bestimmt sein, zu dem mittels des Türsensors ein Schließen der Inkubatortüre erfasst wird. Die Endposition kann insbesondere dadurch bestimmt sein, dass ein in den Bildbereich hineinführender Arm oder eine Hand des Benutzers nicht mehr erfasst wird. Beispielsweise kann ein Bild dahingehend ausgewertet werden, ob ein im Bildrandbereich gelegener, z.B. streifenförmiger, Ausschnitt einem Referenzzustand entspricht, in dem ein Referenzausschnitt der Inkubatorkammer bzw. des Inkubators vollständig sichtbar ist. Ist dies nicht der Fall, kann geschlussfolgert werden, dass ein Benutzer immer noch im Inneren des Inkubators hantiert und das Objekt oder mehrere Objekte immer noch bewegt werden, so dass insbesondere die Videobilderfassung und -auswertung fortzusetzen ist. Die Endposition des Objektes kann als die Position verstanden werden, an der das Objekt nach vorangegangen Positionsänderungen keine Positionsänderung mehr zeigt, kann also durch das Ende der Bewegung des Objektes bestimmt sein. Alternativ oder zusätzlich kann die Endposition des Objektes so definiert sein, dass die Position, die das Objekt inne hat, wenn das Schließen einer Inkubatortüre mittels Türsensor erfasst wird, die Endposition ist. Im Resultat wird die Endposition in den meisten Fällen dieselbe sein.

Die Datenverarbeitungseinrichtung ist vorzugsweise dazu programmiert, die Erfassung des Startbilds und/oder von Videodaten mittels der Kamera zu starten, wenn ein Sensor eine am Inkubator erfolgende Aktivität detektiert. Der Sensor kann ein Bewegungssensor sein, der eine Bewegung in einem außerhalb des Inkubators gelegenen Detektionsbereich detektiert. Der Sensor kann ein Berührungssensor sein, der eine von einem Benutzer durchgeführte Berührung des Inkubators, z.B. eines Türgriffs des Inkubators detektiert. Der Sensor kann ein Türöffnungssensor sein, der insbesondere ein Öffnen einer Inkubatortüre, insbesondere eines Außentüre des Inkubators, erfasst. Der Sensor kann eine Außenkamera des Inkubators sein, die mittels Bildauswertung eine Bewegung und/oder eine Person im Sichtbereich der Kamera erfasst. Der Sensor kann ein Annäherungssensor sein, der, z.B. durch Detektion der Änderung eines elektrischen Feldes, die Annäherung einer Person an den Inkubator erfasst. Eine am Inkubator erfolgende Aktivität kann auch eine Codeeingabe an einem Türschloss sein, die von der Datenverarbeitungseinrichtung vorzugsweise auch ohne Sensor detektierbar ist.

Die Datenverarbeitungseinrichtung ist vorzugsweise dazu programmiert, die Erfassung des Endbilds zu starten und/oder die Erfassung von Videodaten zu stoppen, wenn ein Sensor eine am Inkubator erfolgende Aktivität detektiert. Der Sensor kann ein Türöffnungssensor sein, der insbesondere ein Schließen einer Inkubatortüre, insbesondere eines Außentüre des Inkubators, erfasst. Der Sensor kann eine Außenkamera des Inkubators sein, die mittels Bildauswertung die Beendigung einer Bewegung und/oder das Verschwinden einer Person im Sichtbereich der Kamera erfasst. Der Sensor kann ein Annäherungssensor sein, der, z.B. durch Detektion der Änderung eines elektrischen Feldes, der das Entfernen einer Person vom Inkubator erfasst.

Die Datenverarbeitungseinrichtung ist insbesondere dazu programmiert, die Erfassung von Videodaten mittels der Kamera initiiert vom mittels Türsensor detektierten Öffnen einer Inkubatortüre zu starten. Alternativ oder zusätzlich kann ein Initialereignissensor, insbesondere ein Bewegungssensor, oder Annäherungssensor, oder optischer Sensor/Empfänger (z.B. Lichtschranke), oder Mikrofon, oder Beschleunigungssensor in der Inkubatortüre, im Inkubator angeordnet sein, mittels dem die Annäherung eines Objektes an die Inkubatorkammer oder eines anderen Initialereignisses erfassbar ist; die Datenverarbeitungseinrichtung kann programmiert sein, basierend auf den Daten eines solchen Sensors die Erfassung von Videodaten zu starten. Die Datenverarbeitungseinrichtung kann programmiert sein, mit Vorliegen der Videodaten und/oder mit Vorliegen eines Standbildes die Suche nach einem neuen Objekt in den mittels der Videodaten verfügbaren Bilder (Frames) oder dem Standbild zu beginnen. Alternativ kann die Erfassung von Videodaten starten, sobald ein Benutzer am Inkubator identifiziert wurde, oder bei einem anderen vorgegebenen Ereignis. Auch eine permanente Videodatenerfassung ist möglich. Die Datenverarbeitungseinrichtung ist insbesondere dazu programmiert, die Erfassung von Videobilddaten mit der Erfassung des Endbildes bzw. dem Registrieren der Abwesenheit einer Hand/eines Arms im Kamerasichtbereich zu beenden, oder basierend auf den Ergebnissen eines der genannten Sensoren (Türsensor, Bewegungssensor etc.) zu beenden.

Die Datenverarbeitungseinrichtung ist insbesondere dazu programmiert, die Endposition des mindestens einen Objekts im Lagerbereich in Abhängigkeit von den Identifikationsdaten des mindestens einen Objekts als ID-Positionsdaten in dem Datenspeicher zu speichern. Mit diesem Schritt "kennt" der Inkubator "das Objekt" und dessen Position. Zusammen mit anderen Daten kann er diese Daten nun einem Benutzer ausgeben, insbesondere auf einem Display des Inkubators anzeigen. Zusammen mit Daten über den Eigentümer (der Benutzer der das Objekt in die Inkubatorkammer eingestellt hat) des Objektes oder einen Benutzer des Objektes (z.B. ein Benutzer der ein Bestandsobjekt eines anderen Benutzers bewegt hat) kann der Inkubator diese Datensätze in Abhängigkeit von den Identifikationsdaten des Objektes speichern und sammeln. Die Identifikationsdaten, auf Basis derer die Positionsänderungen detektiert wurden, müssen nicht identisch sein mit den Identifikationsdaten, die als ID-Positionsdaten gespeichert werden; relevant ist, dass die abgespeicherten Identifikationsdaten geeignet sind, das mindestens eine Objekt eindeutig von anderen Objekten bzw. Bestandsobjekten zu unterscheiden. Der ID-Code kann also theoretisch während der Bildverarbeitung wechseln.

Die Zuordnung eines Eigentümers zu einem Objekt kann auf unterschiedliche Weise erfolgen. Vorzugsweise ist die Datenverarbeitungseinrichtung dazu programmiert, den das Objekt in den Inkubator einstellenden Benutzer zu registrieren oder zu identifizieren, diesem Benutzer einen Benutzeridentifikationscode zuzuordnen, und benutzerbezogenen ID-Positionsdaten des Objektes zu speichern. Für eine Nutzerregistrierung kann eine Biometrieerkennung, insbesondere Gesichtserkennung, des Benutzers durchgeführt werden, insbesondere mittels einer Außenkamera, eines Retinascanners oder eines Fingerabdrucksensors des Inkubators. Die entsprechenden registrierten Biometrieerkennungsdaten, insbesondere Gesichtserkennungsdaten des Benutzers können in der Datenspeichereinrichtung des Inkubators, oder in einer externen Datenspeichereinrichtung gespeichert werden. Die Identifizierung des Benutzers kann anhand eines Abgleichs von erfassten Biometrieerkennungsdaten mit bereits registrierten Biometrieerkennungsdaten erfolgen. Alternativ zu einer Biometrieerkennung kann es einem Benutzer auch ermöglicht werden, vor, während oder nach der Durchführung der Objektregistrierung bzw. nach der Ermittlung der Endposition eines verfolgten Objektes einen Benutzeridentifikationsdaten über eine Benutzerschnittstelleneinrichtung einzugeben. Die Benutzerschnittstelleneinrichtung kann eine Tastatur, ein Touchscreen sein und kann Teil des Inkubators oder eines Externgeräts sein.

Ein Vorteil der Objektverfolgung ist, dass diese ohne Kenntnis von Individual- oder Klassen-Merkmalen des zu verfolgenden Objektes durchgeführt werden kann. Sie kann aber auch mit Methoden zur Objekterkennung (und -wiedererkennung) und/oder der Objektklassenerkennung bzw. Klassenwiedererkennung kombiniert werden.

Eine Objekterkennung kann insbesondere als Objektindividualerkennung und/oder als Objektklassenerkennung ausgestaltet sein. Die theoretischen Grundlagen und deren Umsetzung zur praktischen Anwendung in Objekterkennungstechnologien sind bekannt (z.B.: "Deep Learning in Object Detection and Recognition", X. Jiang et al., Springer Singapore, 2019). Algorithmen zur Objekterkennung in Bildern sind allgemein bekannt und verfügbar, z.B. als Bestandteil von OpenCV (zum Beispiel OpenCV 3.3, deep neural network (dnn) module).

Eine Objektindividualerkennung basiert auf der Erkennung objektindividueller Merkmale (Objektindividualmerkmale), anhand der ein Wiedererkennen des individuellen Objektes und ein Unterscheiden gegenüber anderen individuellen Objekten möglich ist. Beispielsweise kann ein Zellkulturbehälter, z.B. ein Einwegprodukt, nachträglich aufgebrachte Individualmerkmale aufweisen, z.B. einen Barcode oder QR-Code. Er kann aber auch über jegliche Merkmale identifizierbar sein, die eine Unterscheidung ermöglichen: z.B. eine Beschriftung, einen unterschiedlichen Inhalt, ein Mikrokratzermuster an der Behälterfläche etc..

Eine Objektklassenerkennung beruht auf der Kenntnis von Objektklassenmerkmalen, die bei der Objektprüfung abgeglichen werden, um dem Objekt eine Klasse zuzuordnen. Beispielsweise kann mit einer Objektklassenerkennung erkannt werden, ob ein Objekt eine bestimmte Art von Zellkulturflasche, eine bestimmte Art von Petrischale oder eine bestimmte Art von Mikrotiterplatte ist, eventuell mit Berücksichtigung weiterer Klassenmerkmale, z.B. Hersteller, Herstellungsjahr, Ausführung, etc..

Der Inkubator weist vorzugsweise ein Objekterkennungssystem auf. Das Objektverfolgungssystem ist vorzugsweise zusätzlich zur Objekterkennung eingerichtet.

Im Falle der Objektindividualerkennung ist eine Datenverarbeitungseinrichtung des Objekterkennungssystems oder des Objektverfolgungssystem vorzugsweise programmiert, a) n einem Standbild, dem Startbild, den Videodaten und/oder dem Endbild Individualmerkmale mindestens einen Objektes zu erkennen, und b) diese Individualmerkmale des Objektes in Form von Objektindividualdaten zu speichern, insbesondere in Abhängigkeit von Identifikationsdaten. Vorzugsweise ist die Datenverarbeitungseinrichtung programmiert, Objektindividualmerkmale des mindestens einen Objektes aus dem Startbild, den Videodaten und/oder dem Endbild zu entnehmen, die Objektindividualmerkmale mit einer Objektindividualdatenbank abzugleichen und, falls die Objektindividualmerkmale in der Objektindividualdatenbank mit einer Objektindividualkennung verbunden sind: die Objektindividualkennung des mindestens einen Objektes zu identifizieren; oder, falls die Objektindividualmerkmale in der Objektindividualdatenbank nicht mit einer Objektindividualkennung verbunden sind: dem mindestens einen Objekt eine Objektindividualkennung zuordnen und in der Objektindividualdatenbank zu speichern, und/oder den ID-Positionsdaten des mindestens einen Objektes die erkannte Objektindividualkennung zuzuordnen und als individualbezogene ID-Positionsdaten zu speichern. Eine Objektindividualkennung ist vorzugsweise unterschiedlich zu dessen Identifikationsdaten; die Objektindividualkennung kann aber auch vorzugsweise gleich zu dessen Identifikationsdaten sein.

Im Falle der Objektklassenerkennung ist eine Datenverarbeitungseinrichtung des Objekterkennungssystems oder des Objektverfolgungssystem vorzugsweise programmiert, a) in einem Standbild, dem Startbild, den Videodaten und/oder dem Endbild Klassenmerkmale mindestens einen Objektes zu erkennen, und b) diese Klassenmerkmale des Objektes in Form von Objektklassendaten zu speichern, insbesondere in Abhängigkeit von Identifikationsdaten. Vorzugsweise ist die Datenverarbeitungseinrichtung programmiert, Objektklassenmerkmale des mindestens einen Objektes in einem Standbild, dem Startbild, den Videodaten und/oder dem Endbild zu erkennen, die Objektklassenmerkmale mit einer Objektklassendatenbank (die insbesondere vorbekannte Korrelationen zwischen der Objektklasse und Objektklassenmerkmalen enthält) abzugleichen und die Objektklasse des mindestens einen Objektes zu erkennen, und insbesondere den ID-Positionsdaten des mindestens einen Objektes die erkannte Objektklasse als Objektklassendaten zuzuordnen und insbesondere als klassenbezogene ID-Positionsdaten zu speichern.

Vorzugsweise weist der Inkubator eine Benutzeridentifikationseinrichtung auf, mittels der ein den Inkubator benutzender Benutzer in Form von Benutzeridentifikationsdaten identifizierbar ist. Vorzugsweise ist eine Datenverarbeitungseinrichtung des Inkubators dazu programmiert, einen den Inkubator benutzenden Benutzer mittels der Benutzeridentifikationseinrichtung zu identifizieren und ihm Benutzeridentifikationsdaten zuzuordnen und Identifikationsdaten und/oder ID-Positionsdaten in Abhängigkeit von den Benutzeridentifikationsdaten als benutzerbezogene Identifikationsdaten und/oder benutzerbezogene ID-Positionsdaten in dem Datenspeicher zu speichern.

Vorzugsweise weist die Benutzeridentifikationseinrichtung eine Außenkamera auf, und vorzugsweise ist die Benutzeridentifikationseinrichtung dazu eingerichtet, und/oder die Datenverarbeitungseinrichtung dazu programmiert, mittels der Außenkamera eine Gesichtserkennung durchzuführen, mittels der der Benutzer identifiziert wird. Vorzugsweise ist eine Benutzerdatenbank vorgesehen, die auf einem Datenspeicher gespeichert ist, der Teil des Inkubators, der Benutzeridentifikationseinrichtung oder des Objektverfolgungssystems sein kann, oder der in einer Datenaustauschverbindung mit der Benutzeridentifikationseinrichtung bzw. der Datenverarbeitungseinrichtung stehen kann, z.B. über ein Intranet oder das Internet. Algorithmen zur Gesichtserkennung in Bildern sind allgemein bekannt und verfügbar, z.B. als Bestandteil von OpenCV ("FaceRecognizer class").

Die Benutzerdatenbank kann eine Korrelation von Benutzeridentifikationsdaten und Benutzermerkmalsdaten enthalten, so dass der Benutzer bzw. dessen Benutzeridentifikationscode (Benutzeridentifikationsdaten) anhand der ermittelten oder eingelesenen Benutzermerkmalsdaten ermittelbar ist. Die Benutzermerkmalsdaten können Informationen über Gesichtsmerkmale der Benutzer enthalten, oder andere biometrische Daten, z.B. Fingerabdruckdaten oder Stimmenerkennungsdaten. Die Benutzerdatenbank kann eine Korrelation von Benutzeridentifikationsdaten und Benutzerkennungen enthalten, wobei die Benutzerkennung ein persönlicher Identifikationscode des Benutzers sein kann, z.B. eine mehrstellige Zeichenfolge, über deren Eingabe an einer Tastatur der Benutzerschnittstelleneinrichtung sich ein Benutzer identifizieren kann.

Die Außenkamera kann insbesondere an, oberhalb oder neben einer Inkubatortüre, insbesondere Außentüre am Inkubator angeordnet bzw. an diesem befestigt sein. Vorzugsweise ist die Außenkamera ein fester Bestandteil der Inkubators oder der Inkubatortüre. Sie kann aber auch über eine Signalverbindung, insbesondere über eine Datenaustauschverbindung, die kabelgebunden oder kabellos sein kann, mit der Benutzeridentifikationseinrichtung bzw. der Datenverarbeitungseinrichtung verbunden sein. Z.B. ist es möglich, die Außenkamera über ein flexibles Kabel mit dem Inkubator bzw. dessen Benutzeridentifikationseinrichtung bzw. der Datenverarbeitungseinrichtung zu verbinden, so dass die Kamera vom Benutzer frei am Inkubator platzierbar ist.

Vorzugsweise weist die Benutzeridentifikationseinrichtung eine Benutzerschnittstelleneinrichtung auf, mittels der Benutzeridentitätsdaten einlesbar sind. Die Benutzerschnittstelleneinrichtung kann eine Tastatur beinhalten, und/oder einen Touchscreen, und oder ein Mikrofon für eine Spracheingabe bzw. zur Realisierung einer Benutzeridentifikation mittels einer Spracherkennung. Die Benutzerschnittstelleneinrichtung kann zum Austausch von Daten mit einem externen datenverarbeitenden Gerät (nachfolgend auch als "Externgerät" bezeichnet) eingerichtet sein. Das Externgerät kann ein PC sein, ein Smartphone, ein Tabletcomputer, oder ein anderer portabler Computer mit Benutzerschnittstelle.

Das Externgerät kann Mittel aufweisen, um einen Benutzer zu identifizieren und/oder zu authentifizieren. Insbesondere aktuell erhältliche Smartphones beinhalten diverse Mittel zur Nutzerauthentifizierung, insbesondere zur Gesichtserkennung. Das Externgerät weist vorzugsweise eine Software auf, z.B. eine App, die dazu programmiert ist, einen Benutzer zu identifizieren und/oder zu authentifizieren, und insbesondere das Ergebnis dieses Vorgangs über die Benutzerschnittstelleneinrichtung an die Benutzeridentifikationseinrichtung des Inkubators zu übersenden. Da ein Externgerät auch oft eine eigene Kamera aufweist, anhand der eine Gesichtserkennung implementiert sein kann, oder einen Fingerabdrucksensor, oder sonstige Hardware zur Benutzeridentifikation und -authentifizierung, sind die entsprechenden Hardwarebestandteile im Fall der Verbindung des Inkubators mit dem Externgerät am Inkubator verzichtbar.

Die Benutzeridentifikationseinrichtung des Inkubators kann als Bestandteil einer Steuerungssoftware des Inkubators programmiert sein. Der Inkubator weist vorzugsweise eine Steuerungseinrichtung auf, die insbesondere eine Datenverarbeitungseinrichtung aufweisen kann, die insbesondere dazu programmiert sein kann, alle oder einige Funktionen der Benutzeridentifikationseinrichtung zu beinhalten, insbesondere den Datenaustausch mit dem Externgerät zu steuern.

Vorzugsweise weist die Benutzeridentifikationseinrichtung eine Benutzerschnittstelleneinrichtung auf, mittels der Benutzeridentitätsdaten auswählbar sind. Dazu kann insbesondere die Benutzeridentifikationseinrichtung ein Display bzw. einen Touchscreen aufweisen, über das eine Liste möglicher Benutzer, z.B. über die Angabe eines Namens oder Bildes des Benutzers, anzeigbar ist. Es können dann Eingabemittel vorgesehen sein, z.B. Tasten, eine Tastatur, Touchpad, der Touchscreen, über die der Benutzer die Auswahl aus der Liste vornehmen kann.

Die Benutzeridentifikationseinrichtung kann dazu programmiert sein, eine Benutzerauthentifizierung vorzunehmen, indem das genannte Einlesen der Benutzeridentitätsdaten oder die genannte Auswahl aus der Liste passwortgeschützt ist, so dass der Benutzer erst als identifiziert gilt, wenn die Authentifizierung erfolgreich war.

Vorzugsweise weist die Benutzeridentifikationseinrichtung ein Lesegerät zum Einlesen eines den Benutzer identifizierenden Codes auf, wobei das Lesegerät insbesondere ein RFID-Lesegerät, ein Barcode-Lesegerät, oder ein QR-Code-Lesegerät ist.

Die Benutzeridentifikationseinrichtung bzw. eine/deren Datenverarbeitungseinrichtung kann dazu programmiert sein, eine verriegelte Inkubatortüre in Abhängigkeit von der Benutzeridentifikation zu entriegeln und/oder zu verriegeln, insbesondere eine verriegelte Inkubatortüre zu entriegeln, wenn der Benutzer erfolgreich identifiziert wurde. Dies bedeutet in diesem Fall, dass der Benutzer auch für den Inkubator zugangsberechtigt ist. Es kann aber auch eine zusätzliche Zugangsrechteliste geben, anhand derer der Inkubator entscheidet, ob ein identifizierter Benutzer das Zugangsrecht besitzt oder, ggf. Welche Art Zugangsrecht der identifizierte Benutzer besitzt. Das Zugangsrecht kann z.B. beschränkt sein auf bestimmte Zeiten, insbesondere Wochentage, Uhrzeiten, oder Berechtigungszeitabschnitte. Wenn der Inkubator mehrere Inkubatortüren aufweist, kann das Zugangsrecht vorsehen, dass der Benutzer nur für eine vorbestimmte Auswahl dieser Inkubatortüren das Zugangsrecht besitzt.

Vorzugsweise weist der Inkubator genau eine -oder auch mehrere- Inkubatortüre(n) zum Verschließen der Kammeröffnung auf. Die Inkubatortüre bildet im verschlossenen Zustand insbesondere einen Teil des Inkubatorgehäuses, dem das als thermischer Isolator der Inkubatorkammer des Inkubators dient. Die Inkubatortüre kann an ihrer Außenseite eine Benutzerschnittstelleneinrichtung aufweisen, insbesondere ein Display. Eine Datenverarbeitungseinrichtung des Inkubators bzw. der Benutzerschnittstelleneinrichtung kann dazu programmiert sein, ein von der Kamera des Inkubators aufgenommenes Bild des mindestens einen Lagerbereichs des Inkubators anzuzeigen.

Vorzugsweise weist der Inkubator einen Türsensor zum Erfassen des Öffnens bzw. des Schließens der Inkubatortüre auf. Vorzugsweise ist die Datenverarbeitungseinrichtung dazu programmiert, die Überwachung des Innenraums des Inkubators, insbesondere die Generierung der Videodaten und/oder Standbilddaten, in Abhängigkeit von der Detektion einer Türöffnung des Inkubators zu starten, insbesondere mittels eines Türsensors, alternativ auch mittels eines Bewegungssensor oder Annäherungsdetektors.
vorzugsweise ist die Datenverarbeitungseinrichtung dazu programmiert, die Überwachung des Innenraums des Inkubators, insbesondere die Generierung der Videodaten und/oder Standbilddaten zu starten, insbesondere in Abhängigkeit von der Detektion einer Türöffnung durch einen Benutzer, der mittels einer Benutzeridentifikationseinrichtung identifiziert wurde;
vorzugsweise ist die Datenverarbeitungseinrichtung dazu programmiert die Überwachung des Innenraums des Inkubators, insbesondere die Generierung der Videodaten, in Abhängigkeit von der Detektion einer Türschließung oder in Abhängigkeit von der Messung eines der genannten Sensoren des Inkubators zu beenden;
insbesondere ist die Datenverarbeitungseinrichtung dazu programmiert mittels der Informationen aus der Benutzeridentifikationseinrichtung und der Objektverfolgungseinrichtung festzustellen, durch welchen Benutzer welches Objekt im Innenraum bewegt wurde, und die Benutzeridentifikationsdaten dieses Benutzers gemeinsam mit den Objektsidentifikationsdaten dieses Objektes in dem Datenspeicher zu speichern.

Vorzugsweise ist die Datenverarbeitungseinrichtung dazu programmiert, aus dem Startbild, den Videodaten und/oder dem Endbild den Bewegungspfad, des mindestens einen Objektes innerhalb der Inkubatorkammer zu ermitteln und in Form von Bewegungshistoriedaten in dem Datenspeicher zu speichern, insbesondere zeitabhängig zu speichern. Vorzugsweise ist die Datenverarbeitungseinrichtung dazu programmiert, aus dem Startbild, den Videodaten und/oder dem Endbild eine Bewegungshistorie des mindestens einen Objektes innerhalb der Inkubatorkammer zu ermitteln und in Form von Bewegungshistoriedaten in dem Datenspeicher zu speichern, insbesondere zeitabhängig zu speichern, vorzugsweise mit Informationen über Anzahl und/oder Zeitpunkte der mittels Türsensor ermittelten Änderungen des Status der Türöffnung (offen/geschlossen) der Inkubatortüre. Der Bewegungspfad enthält vorzugsweise gespeicherte Positionsdaten des Objektes, wobei diese Positionsdaten den Bewegungspfad des Objektes markieren, insbesondere zwischen einem Startbild und einem Endbild, insbesondere zwischen einer -insbesondere unbewegten oder auch bewegten- Startposition des Objektes und einer -insbesondere unbewegten- Endposition. Die Bewegungshistoriedaten enthalten vorzugsweise zeitabhängig gespeicherte Positionsdaten bzw. Bewegungspfade, vorzugsweise innerhalb mindestens eines Zeitabschnitts oder während des gesamten Aufenthaltes dieses Objektes im Inkubator. Bewegungshistoriedaten können auch Informationen zu dem die Positionsänderung auslösenden Benutzer in Form von Benutzeridentifikationsdaten umfassen. Dies ist insbesondere bei Objekten, die wertvolle Proben enthalten, von Vorteil.

Vorzugsweise weist der Inkubator ein Display (= einen Bildschirm) auf. Der Bildschirm ist vorzugsweise ein fester Bestandteil des Inkubators, insbesondere der Inkubatortüre. Er kann aber auch entfernt vom Inkubator angeordnet sein und kann insbesondere Bestandteil eines Externgerätes sein, das in einer Datenaustauschverbindung mit der Datenverarbeitungseinrichtung des Inkubators stehen kann.

Vorzugsweise ist die Datenverarbeitungseinrichtung dazu programmiert, eine graphische Wiedergabe des Innenraums der Inkubatorkammer, insbesondere des mindestens einen Lagerbereichs, auf dem Bildschirm anzuzeigen. Die graphische Wiedergabe kann ein Foto des Lagerbereichs beinhalten, auf dem ein oder mehrere Bestandsobjekte des Inkubators angezeigt sein können. Der Lagerbereich kann insbesondere ein Regalbrett im Inkubator sein oder ein vorbestimmter Ausschnitt desselben. Das Foto kann ein mit der Kamera aufgenommenes Bild zeigen, dass gegebenenfalls nachbearbeitet sein kann. Vorzugsweise beinhaltet diese Nachbearbeitung, dass ein von der Kamera verzerrt aufgenommenes Bild begradigt wird. Algorithmen für solch eine Nachbearbeitung sind allgemein bekannt und frei verfügbar (for example: OpenCV, "Omnidirectional Camera Calibration"). Die Verzerrung kann insbesondere optisch bedingt sein und auf die Verwendung einer Weitwinkel- bzw. Fischaugenkamera zurückzuführen sein.

Die graphische Wiedergabe kann insbesondere eine abstrahierte Darstellung eines von der Kamera aufgenommenen Bildes oder Bildausschnitts sein. Beispielsweise kann die graphische Wiedergabe eine aus der Vogelperspektive (oder einer anderen Perspektive) gezeigter abstrahierter Lagerbereich sein, insbesondere die graphische Darstellung eines Rechtecks oder die perspektivische Darstellung eines Quaders. Die Bestandsobjekte können ebenfalls in abstrahierter Form dargestellt sein, z.B. als rechteckförmige oder quaderförmige graphische Bildobjekte. Ziel einer solchen Darstellung ist es insbesondere, dem Benutzer über den Standort des Objektes/der Objekte im Inkubator bzw. im Lagerbereich zu informieren. Dies ermöglicht einen schnellen Zugriff des Benutzers auf das/die gewünschte(n) Objekt(e) und minimiert die Zeit, in der die Inkubatortüre geöffnet ist. Im Fall, dass die Differenzierung zwischen in einem Stapel aus Objekten enthaltenen Einzelobjekten ermöglicht werden soll, ist eine graphische Wiedergabe aus einer von der Vogelperspektive abweichenden Perspektive sinnvoll, beispielsweise aus einer seitlichen Perspektive, um einzelne Objekte eines Stapels graphisch hervorheben zu können.

Vorzugsweise ist die Datenverarbeitungseinrichtung dazu programmiert, graphisch anzuzeigen, wo das durch die Objektpositionsdaten, also die ID-Positionsdaten, identifizierte Objekt im Lagerbereich bzw. Im Innenraum der Inkubatorkammer positioniert ist, bzw. um graphisch anzuzeigen, wo alle im Innenraum lokalisierten Objekte angeordnet sind.

Vorzugsweise ist die Datenverarbeitungseinrichtung dazu programmiert, in Abhängigkeit von mindestens einem Bedingungsparameter ein Objekt oder mehrere Objekte im Display graphisch hervorzuheben. Der Bedingungsparameter kann die Benutzeridentifikationsdaten bezeichnen:
Vorzugsweise ist die Datenverarbeitungseinrichtung dazu programmiert, in Abhängigkeit von Benutzeridentifikationsdaten eines Benutzers (Einzelnutzer, einer Nutzergruppe, oder mehrere Benutzer) ein Objekt oder mehrere Objekte graphisch hervorzuheben, das dem Benutzer als Eigentum zugeordnet ist, nämlich z.B. indem die benutzerbezogenen ID-Positionsdaten die Benutzeridentifikationsdaten dieses Benutzers enthalten. Eigentümer ist derjenige, der das Objekt betreut und -in den meisten Fällen selber oder mithilfe einer Hilfsperson- in die Inkubatorkammer eingestellt hat. Vorzugsweise ist die Datenverarbeitungseinrichtung dazu programmiert, ausgehend von vorgegebenen Benutzeridentifikationsdaten festzustellen, wo die diesen Benutzeridentifikationsdaten mittels der benutzerbezogenen Objektpositionsdaten zugeordneten Objekte positioniert sind und, insbesondere, diese Objekte graphisch hervorzuheben.

Der Bedingungsparameter kann auch Informationen über eine Zeitdauer oder einen Zeitpunkt beinhalten, z.B. die Zeitdauer mit der ein Objekt bereits in der Inkubatorkammer angeordnet war. Dadurch kann ein Benutzer sich schnell einen Überblick darüber verschaffen, wie lange ein oder mehrere Objekte bereits in der Inkubatorkammer lagern, eventuell dort von ihrem Eigentümer vergessen wurden. Oder der Inkubator kann abhängig von einem mittels eines Sensors des Inkubators detektierten Ereignisses oder abhängig von einem Zeitplan, der im Inkubator oder einem Externgerät gespeichert sein kann, ein oder mehrere Objekte graphisch hervorheben, die der Aufmerksamkeit des Benutzers bzw. des Laborpersonals bedürfen.

Oder der Bedingungsparameter kann, im Falle der Implementierung einer Objektklassenerkennung, Informationen über eine bestimmte Objektklasse beinhalten. Es können so ein oder mehrere Objekte derselben Objektklasse (oder der davon abweichenden Objektklassen) graphisch hervorgehoben werden, beispielsweise um den Standort aller Petrischalen (und nicht: Zellkulturflaschen) im Inkubatorinnenraum hervorzuheben.

Oder der Bedingungsparameter kann, im Falle der Implementierung einer Objektindividualerkennung, Informationen über ein bestimmtes Objektindividuum beinhalten. Auf diesem Weg lässt sich eine Objektsuche anhand von Individualmerkmalen umsetzen, indem z.B. der Inkubator Mittel zur Eingabe von Individualmerkmalen aufweist, z.B. einen Barcode, QR-Code, eine individuelle Beschriftung oder ein Foto des individuellen Objektes. Es kann so das individuelle Objekt graphisch hervorgehoben und leicht gefunden werden.

Vorzugsweise ist die Datenverarbeitungseinrichtung dazu programmiert, eine graphische Wiedergabe des Innenraums der Inkubatorkammer, insbesondere des mindestens einen Lagerbereichs, auf dem Bildschirm anzuzeigen und insbesondere, den im Inkubator verfügbaren freien Lagerplatz graphisch anzuzeigen bzw. hervorzuheben. Beispielsweise kann der Lagerbereich abstrahiert gezeigt werden und eine freie Lagerposition (oder mehrere verfügbare freie Lagerplätze) können graphisch hervorgehoben sein, indem der entsprechende Bereich z.B. in grüner oder weißer, oder einer zeitlich wechselnden (Blinken), Kontrastfarbe zum Hintergrund dargestellt wird. Auf diese Weise muss der Benutzer keine Zeit darauf verwenden, einen möglichen freien Lagerplatz zu suchen, oder einen solchen durch Bewegung von Bestandsobjekten zu schaffen.

Zudem kann, ähnlich der Funktion eines Parkwächters, die Datenverarbeitungseinrichtung dazu programmiert sein, die Belegung des Innenraums der Inkubatorkammer bzw. des mindestens einen Lagerbereichs zu planen und insbesondere auf diese Weise die Nutzung der verfügbaren Lagerfläche zu optimieren. Dazu kann die Datenverarbeitungseinrichtung dazu programmiert sein, vorbestimmte Abstände zwischen einem oder mehreren Bestandsobjekte zu einem neu einzustellenden Objekt zu berücksichtigen und insbesondere dem Benutzer vorzuschlagen, indem der freie Lagerplatz entsprechend als verfügbar und/oder nicht verfügbar hervorgehoben angezeigt wird. Gemäß dieser Beispiele kann der Inkubator ein computer-/softwareimplementiertes Planungsprogramm zur Belegung des Innenraums des Inkubators aufweisen, das insbesondere die Position mindestens eines Objekts im Innenraum (Bestandsobjekts) und/oder insbesondere den frei verfügbaren Lagerplatz berücksichtigt, eventuell auch die Zeitpunkte zu denen das mindestens eine Bestandsobjekt neu eingestellt wurde, oder Zeitpunkte in der Zukunft, an denen das Einstellen weiterer Objekte in den Inkubator geplant sind. Solche Zeitpunkte können insbesondere bekannt sein, wenn der Inkubator mit einem Laborinformationssystem (LIS) oder einem anderen (Labor-)Datenaustauschnetzwerk verbunden ist. Der Inkubator weist vorzugsweise einen Zeitgeber auf, eine Uhr, oder einen Zeitmesser.

Es ist aber auch möglich und besonders bevorzugt, dass die Datenverarbeitungseinrichtung des Inkubators (nachfolgend: "erste Datenverarbeitungseinrichtung") die Durchführung einer Messung mindestens eines Messwertes steuert, um insbesondere eine die Atmosphäre im Innenraum der Inkubatorkammer kennzeichnenden Parameter (Temperatur, Gaspartialdruck) zu messen. Auf diese Weise kann beim Betrieb des Objektverfolgungssystems von der ersten Datenverarbeitungseinrichtung berücksichtigt werden, dass der Betrieb von elektrischen Einrichtungen innerhalb der Inkubatorkammer eventuell zu Abwärme führt, welche die Kammeratmosphäre in inakzeptablem Maße erwärmt. Die Steuereinrichtung des Inkubators kann deshalb insbesondere dazu eingerichtet sein, den Betrieb der elektrischen Einrichtungen, insbesondere der mindestens einen Kamera innerhalb der Inkubatorkammer in Abhängigkeit von mittels Temperatursensoren erfassten Temperaturen der Kammeratmosphäre zu steuern. Die Steuereinrichtung des Inkubators kann insbesondere dazu eingerichtet sein, die Temperierung der Kammeratmosphäre mittels der mindestens einen Temperiereinrichtung des Inkubators und den Betrieb der elektrischen Einrichtungen innerhalb der Inkubatorkammer in Abhängigkeit voneinander zu steuern, um die unerwünschte Erwärmung der Kammeratmosphäre zu kompensieren.

Es ist möglich und besonders bevorzugt, dass eine Datenverarbeitungseinrichtung des Inkubators dazu programmiert ist, einen Belegungszustand des Innenraums der Inkubatorkammer zu ermitteln, und/oder vorzugsweise zu Durchführung einer oder mehrerer der folgenden Schritte programmiert ist, insbesondere in Abhängigkeit von den ID-Positionsdaten des mindestens einen im Innenraum angeordneten Objektes einen Belegungszustand des Innenraums der Inkubatorkammer zu ermitteln, in Abhängigkeit von den klassenbezogene ID-Positionsdaten des mindestens einen im Innenraum angeordneten Objektes einen Belegungszustand des Innenraums der Inkubatorkammer zu ermitteln, in Abhängigkeit von den individualbezogenen ID-Positionsdaten des mindestens einen im Innenraum angeordneten Objektes einen Belegungszustand des Innenraums der Inkubatorkammer zu ermitteln.

Ein Belegungszustand des Innenraums kann durch Informationen definiert sein, die das im Innenraum von dem mindestens einen Objekt eingenommene Volumen beschreiben, und/oder die das im Innenraum von dem mindestens einen Objekt nicht eingenommene Volumen, also das freie Volumen beschreiben, und/oder die von dem mindestens einen Objekt eingenommene Lagerfläche auf mindestens einem Lagerbereich bzw. auf dem insgesamt verfügbaren Lagerbereich im Innenraum der Inkubatorkammer, und/oder die von dem mindestens einen Objekt nicht eingenommene, also die freie, Lagerfläche auf mindestens einem Lagerbereich bzw. auf dem insgesamt verfügbaren Lagerbereich im Innenraum der Inkubatorkammer, wobei diese Angaben jeweils auf das gesamte Innenraumvolumen bzw. die gesamte Lagerfläche bezogen sein können, wobei diese Informationen z.B. das Verhältnis eines nicht verfügbaren (belegten) oder eines freien (nicht belegten) Innenraumvolumens zum Gesamtvolumen des Innenraums beinhalten können, oder wobei diese Informationen z.B. das Verhältnis einer nicht verfügbaren (belegten) oder einer freien (nicht belegten) Lagerfläche zur Gesamtlagerfläche im Innenraum beinhalten können.

Belegungszustandsdaten, welche die Informationen über den Belegungszustand enthalten, können auch die ID-Positionsdaten, klassenbezogene ID-Positionsdaten und/oder individualbezogene ID-Positionsdaten enthalten. Auf diese Weise ist die Angabe einer Ortsauflösung der Belegung möglich, also die Angabe zur Lokalisation der Belegung im Innenraum, bzw. eine Dichteverteilung der Objekte im Innenraum.

Es ist möglich und besonders bevorzugt, dass eine Datenverarbeitungseinrichtung des Inkubators dazu programmiert ist, Informationen über den Belegungszustand des Inkubators in Form von Belegungszustandsdaten in einem Datenspeicher zu speichern, insbesondere auf ein externes Datenverarbeitungsgerät, insbesondere eines Laborgeräts, eines PC's, oder eines mobilen Computers, insbesondere eines Tabletcomputers oder eines Smartphones zu übertragen. Diese Informationen sind insbesondere abrufbar gespeichert, insbesondere abrufbar vom Benutzer mittels einer Benutzerschnittstelleneinrichtung.

Es ist möglich und besonders bevorzugt, dass eine Datenverarbeitungseinrichtung des Inkubators dazu programmiert ist, Informationen über den Belegungszustand des Inkubators auf einem Bildschirm des Inkubators oder eines externen Datenverarbeitungsgeräts anzuzeigen, insbesondere in Abhängigkeit von Belegungszustandsdaten, die einem Datenspeicher entnommen sein können. Das externe Datenverarbeitungsgerät kann Teil eines Laborgeräts, PC's, eines mobilen Computers, insbesondere eines Tabletcomputers oder eines Smartphones sein.

In Testserien, die Ausführungsformen der vorliegenden Erfindung zugrunde liegen, wurde festgestellt, dass der sich nach einer Öffnung der Inkubatortüre aufgrund der Temperaturregelung ergebende zeitliche Temperaturverlauf in der Inkubatorkammer von dem Belegungszustand der Inkubatorkammer abhängt. Ist ein größeres Volumen des Kammerinnenraums durch Bestandsobjekte belegt, so liegt ein geringeres freies Kammerinnenraumvolumen vor, dass sich aus der Differenz des Kammerinnenraums und des von den Objekten eingenommenen Belegungsvolumens ergibt. Eine auf die Regelung des gesamten Innenraumvolumens ausgelegte Temperaturregelung wird in dieser Situation eventuell andere, nicht erwünschte Resultate erzielen. Es kann zu einem raschen Überschwingen kommen, das unerwünscht ist, auch wenn dabei die Wiederherstellung der Zieltemperatur, z.B. 37 °C, beschleunigt sein kann, d.h. auch wenn sich die Erholungszeit dabei verkürzt. Wenn mehrere neue Objekte mit niedrigeren Temperaturen als der Zieltemperatur neu eingestellt werden, kann es auch zu einer Verzögerung der Erholungszeit kommen, die Kenntnis über kältere, neu eingestellte Objekte kann aber ebenfalls zu einer Anpassung der Temperaturregelung verwendet werden. Die Temperaturregelung der Temperatur im Innenraum der Inkubatorkammer hängt von Regelungsparametern ab.

Vorzugsweise ist eine elektronische Steuereinrichtung des Inkubators dazu eingerichtet bzw. programmiert, dass mindestens eine Temperiereinrichtung des Inkubators, die zur Temperierung der Inkubatorkammer angeordnet ist, während der Temperaturregelung in Abhängigkeit von der Zeit t mit der elektrischen Leistung Pₜₑₘₚ(t) betrieben wird. Insbesondere kann der Inkubator dazu eingerichtet sein, dass die Temperiereinrichtung mittels einer Pulsweitenmodulation (PWM) des Stroms betrieben wird. Die Leistung wird dann insbesondere durch den Tastgrad der PWM bestimmt, da die Amplitude des Stroms vorzugsweise konstant ist. Insbesondere die genannten Größen können Variablen der Temperaturregelung sein, also Regelungsparameter.

Vorzugsweise ist eine elektronische Steuereinrichtung des Inkubators dazu eingerichtet bzw. programmiert, dass die Temperaturregelung oder die Regelung der Inkubatorgaszuführung (z.B. CO₂, N₂, und/oder O₂), insbesondere mindestens ein Regelungsparameter, in Abhängigkeit vom Belegungszustand der Inkubators angepasst werden kann. Auf diese Weise lässt sich der Einfluss von im Innenraum der Inkubatorkammer angeordneten Objekten auf das Ansprechverhaltendes geregelten Systems berücksichtigen. Insbesondere lässt sich die Erholungszeit im Falle einer größeren Belegung des Innenraums reduzieren.

Die Datenverarbeitungseinrichtung des Objektverfolgungssystems ist vorzugsweise separat zu einer ersten Datenverarbeitungseinrichtung des Inkubators. Sie kann aber auch Bestandteil der Steuereinrichtung des Inkubators (auch bezeichnet als "erste Steuereinrichtung") sein, die Funktionen des Inkubators steuert. Die Funktionen der Steuereinrichtung sind insbesondere durch elektronische Schaltkreise implementiert. Die Datenverarbeitungseinrichtung des Objektverfolgungssystems kann mindestens eine CPU, und/oder mindestens eine GPU aufweisen. Eine GPU kann zur Bildverarbeitung oder der Durchführung von Deep Learning-Prozessen vorgesehen sein. Alternativ zu einer CPU, oder einer GPU kann die Datenverarbeitungseinrichtung auch einen dedizierten Chip, z.B. den Nvidia Jetson, zur Bildverarbeitung oder der Durchführung von Deep Learning-Prozessen aufweisen, die vorzugsweise bei der Objektverfolgung, insbesondere bei einer möglichen Objektklassifizierung oder Objektindividualerkennung verwendet werden können. Solche dedizierten Chips kann man als Rechenbeschleuniger zur Datenverarbeitungseinrichtung hinzufügen. Eine GPU ist auf vielen System on a Chip Systemen (SoC) schon vorhanden (für die Widergabe von Grafik und Videos). Ein Raspberry PI kann ebenfalls als Teil des SOCs eine dedizierte GPU Einheit aufweisen. Die Datenspeichereinrichtung weist vorzugsweise mindestens einen Datenspeicher auf, der insbesondere ein flüchtiger oder ein nicht-flüchtiger Datenspeicher sein kann. Die vom Inkubator erfassten oder empfangenen Daten sind auf diesem mindestens einen Datenspeicher speicherbar, insbesondere in mindestens einer Datenbank, die in mindestens einem Datenspeicher gespeichert sein kann. Zu diesen Daten gehören insbesondere mindestens eine oder alle der folgenden Datenarten: Identifikationsdaten, ID-Positionsdaten, Benutzeridentifikationsdaten, benutzerbezogene ID-Positionsdaten, Objektsidentifikationsdaten, Bewegungshistoriedaten, klassenbezogene ID-Positionsdaten, individualbezogene ID-Positionsdaten, Belegungszustandsdaten, Objektdaten, Bilddaten, Standbilddaten, Videobilddaten. Die Datenspeichereinrichtung ist vorzugsweise Bestandteil des Inkubators, also insbesondere in einem Gehäuse des Inkubators angeordnet. Sie kann aber auch Bestandteil eines externen Datenverarbeitungsgeräts sein, mit dem der Inkubator bzw. dessen Datenverarbeitungseinrichtung kommuniziert.

Das Objektverfolgungssystem kann eine Steuerungseinrichtung aufweisen, die separat zur ersten Steuereinrichtung vorgesehen sein kann. Die Begriffe "Steuereinrichtung" und "Steuerungseinrichtung" werden in dieser Beschreibung synonym verwendet. Eine Steuereinrichtung kann einen Mikroprozessor aufweisen, der die Datenverarbeitungseinrichtung beinhalten kann. Der Mikroprozessor kann vom Typ "Raspbery Pi" sein. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird -jeweils bezogen auf den Inkubator und/oder das Objektverfolgungssystem. Die Funktionen des Inkubators und/oder des Objektverfolgungssystems und/oder der Steuereinrichtung und/oder der Datenverarbeitungseinrichtung können in Verfahrensschritten beschrieben werden. Sie können als Bestandteile des Steuerungsprogramms realisiert sein, insbesondere als Unterprogramme des Steuerungsprogramms.

Eine Steuereinrichtung weist im Rahmen der vorliegenden Erfindung generell insbesondere die Datenverarbeitungseinrichtung, insbesondere eine Recheneinheit (CPU) zum Verarbeiten von Daten und/oder einen Mikroprozessor auf oder ist die Datenverarbeitungseinrichtung. Die Datenverarbeitungseinrichtung der Steuereinrichtung des Inkubators kann vorzugsweise auch zum Steuern des Objektverfolgungssystems eingerichtet sein.

Die Datenverarbeitungseinrichtung des Objektverfolgungssystems ist vorzugsweise eine außerhalb der Inkubatorkammer oder des Inkubators und insbesondere optional getrennt von dieser/diesem angeordnete Vorrichtung, auch als externe Vorrichtung bzw. externe Datenverarbeitungseinrichtung bezeichnet. Die Datenverarbeitungseinrichtung und der Inkubator stehen vorzugsweise in einer Datenverbindung und sind vorzugsweise Bestandteile eines Netzwerks zum Datenaustausch.

Die mindestens eine Kamera des Objektverfolgungssystems ist vorzugsweise über eine Kabelverbindung mit der Steuereinrichtung bzw. Datenverarbeitungseinrichtung des Objektverfolgungssystems verbunden. Dazu weist die Inkubatorkammer eine Durchgangsöffnung auf, durch die das Kabel der Kabelverbindung geführt ist. Dabei ist vorzugsweise eine Dichtung, insbesondere Silikondichtung vorgesehen, die den Port abdichtet, um eine Beeinflussung der Atmosphäre im Inkubator (weitestgehend) zu verhindern. Alternativ ist die Kamera für einen kabellosen Datenaustausch mit der Steuereinrichtung bzw. Datenverarbeitungseinrichtung verbunden, z.B. per Bluetooth oder WLAN.

Der Inkubator kann ein Teilgehäuse aufweisen, in dem insbesondere mindestens eine Steuerungseinrichtung (des Inkubators und/oder des Objektverfolgungssystems) angeordnet ist. Das Teilgehäuse ist vorzugsweise an der Rückseite des Inkubators, also insbesondere gegenüberliegend der Inkubatortüre angeordnet.

Das System, der Inkubator und/oder das des Objektverfolgungssystem und/oder die Datenverarbeitungseinrichtung und/oder die Steuerungseinrichtung sind vorzugsweise dazu eingerichtet, die Positionsdaten des mindestens einen Objektes oder einer Vielzahl von Objekten zur Bildung einer elektronischen Dokumentationsdatei zu verwenden, in der die Bewegung der Objekte protokolliert und dokumentiert wird. Diese Dokumentationsdatei wird dann insbesondere in einer Datenspeichereinrichtung gespeichert und vorzugsweise laufend aktualisiert. Auf diese Weise kann ein "korrekter" Umgang mit den Objekten gemäß Standartprotokollen bei Bedarf zertifiziert werden. Andererseits können nachträglich Abweichungen von Standartprotokollen ermittelt werden und/oder Informationskorrelationen ermittelt werden. Durch die Erfassung solcher Daten kann die Qualität von zellbasierten Laborarbeiten bzw. medizinischen, biologischen und pharmazeutischen Verfahren erheblich verbessert werden und zuverlässiger werden. Die Reproduzierbarkeit der zell-basierten Laborarbeiten kann erhöht werden, Abweichungen von normalen Eigenschaften können frühzeitige erkannt werden, um dem Anwender die Möglichkeit einer Korrektur oder frühzeitigen Wiederholung des Experiments zu geben. Die Dokumentationsdatei kann dem Benutzer oder einer externen Datenverarbeitungseinrichtung von der Steuerungseinrichtung über Datenaustausch bereitgestellt werden. Insbesondere in kritischen Anwendungen, die z.B. einen forensischen Bezug haben oder bei denen Zellen von erheblichen Wert kultiviert werden, ist eine solche Dokumentation besonders sinnvoll.

Der Inkubator ist ein Labor-Inkubator und damit ein Gerät, mit dem kontrollierte Klimabedingungen für verschiedene biologische Entwicklungs- und Wachstumsprozesse geschaffen und erhalten werden können. Der Inkubator kann vorzugsweise ein Shaker, also ein Inkubator mit einer Bewegungseinrichtung zur Bewegung von in der Inkubatorkammer angeordneten Objekten, sein oder diesen beinhalten. Der Inkubator kann eine Zellkultivierungsvorrichtung sein, ein Microbial incubator (auch ohne CO₂). Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N₂-Gehalts Gehalts des Gases und/oder eine Einstelleinrichtung zur Einstellung der Luftfeuchtigkeit in der Inkubatorkammer des Inkubators. Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden. Eine mit Wasser gefüllte Wanne in der Inkubatorkammer kann geheizt oder gekühlt werden, um über die Verdunstung die Luftfeuchtigkeit einzustellen. CO₂-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen. Der erfindungsgemäße Inkubator ist insbesondere nicht ein Bioreaktor und/oder Fermentor.

Eine Sensoreinrichtung weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Ein Inkubator weist vorzugsweise eine bzw. eine einzige Inkubatorkammer auf. Diese kann in Kompartimente unterteilt sein. Kompartimente können durch -insbesondere gelochte- Lagerplatten getrennt sein, wobei insbesondere ein Gasaustausch zwischen den Kompartimenten ermöglicht ist. Eine Lagerplatte, insbesondere deren untere Seite, kann zum Halten der Kameraeinrichtung eingerichtet sein und kann insbesondere eine Halterung für die Kameraeinrichtung aufweisen. Eine Lagerplatte, insbesondere deren untere Seite, kann zum Halten der Beleuchtungseinrichtung eingerichtet sein und kann insbesondere eine Halterung für die Beleuchtungseinrichtung aufweisen.

Die Inkubatorkammer weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Diese Kammeröffnung ist durch ein mit der Inkubatorkammer beweglich verbundenes Verschlusselement verschließbar, insbesondere eine mittels Scharnier an der Inkubatorkammer beweglich montierte Inkubatortüre, insbesondere eine oder mehrere Kammertüren. Ein Inkubator kann eine oder mehrere Innentüren aufweisen, die insbesondere transparent sein können, und kann eine Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert. Vorzugsweise werden vom Bilderfassungssystem Bilder bei geschlossener Inkubatortüre bzw. Außentüre erfasst, damit ein Umgebungslicht keinen Einfluss auf die Beleuchtung des Lagerbereichs nimmt, die vorzugsweise ausschließlich durch die Beleuchtungseinrichtung erfolgt. Dies führt zu besonders gut reproduzierbaren, gut vergleichbaren und einfach durch Bildverarbeitungsalgorithmen auswertbaren Bildaufnahmen. Dennoch ist es auch möglich, dass die Bildaufnahmen bei geöffneter Inkubatortüre, insbesondere Außen- und/oder Innentüre erstellt werden.

In der verschlossenen Position der Kammeröffnung ist das Innere der Inkubatorkammer gegen die Umgebung vorzugsweise in der Weise isoliert, so dass im Inneren eine gewünschte, vom Inkubator gesteuerte Atmosphäre einstellbar, insbesondere regelbar ist. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, Kupfer, Messing, oder ein Kunststoff, insbesondere ein Verbundkunststoff. Dadurch wird die Reinigung/ Desinfektion des Kammerinneren erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Kabelverbindung aus dem Inneren der Inkubatorkammer an deren Außenseite oder in die Umgebung des Inkubators aufweisen.

Vorzugsweise sind die Oberflächen der Inkubatorinnenwände nicht-glänzend bzw. nicht-reflektierend ausgestaltet, insbesondere durch Verwendung einer matten Oberfläche. Die Oberfläche der Inkubatorinnenwand kann durch eine Oberflächenbehandlung mattiert sein. Die Oberflächenbehandlung kann insbesondere Schleifen mit einem Schleifmittel sein, das insbesondere eine bestimmte Körnung aufweisen kann. Die Oberflächenbehandlung kann insbesondere Bestrahlen mit einem Strahlmittel, insbesondere Sand oder Glasperlen insbesondere per Druckluft, sein, das insbesondere eine bestimmte Körnung bzw. einen charakteristischen Partikeldurchmesser aufweisen kann. Dadurch können störende Reflexionen in einer Bildaufnahme verhindert bzw. reduziert werden.

Eine typische Größe des Inneren einer Inkubatorkammer liegt zwischen 50 und 400 Litern.

Der Inkubator kann genau eine Inkubatorkammer aufweisen, kann aber auch mehrere Inkubatorkammern aufweisen, deren Atmosphäre (Temperatur, relative Gaskonzentration, Luftfeuchte) insbesondere individuell oder gesammelt einstellbar sein kann. Ein Inkubator kann mehrere Inkubatorkammern aufweisen, die jeweils eine eigene Kammeröffnung und eine eigene Kammertüre zum Verschließen der Kammeröffnung aufweisen können.

Der Inkubator kann ein Gehäuse aufweisen, dass die Inkubatorkammer teilweise oder vollständig umgibt. Das Gehäuse kann im Wesentlichen quaderförmig ausgebildet sein, und kann insbesondere derart ausgebildet sein, dass der Inkubator stapelbar ist.

Ein Lagerbereich des Inkubators ist insbesondere durch eine Lagerplatte, insbesondere einen Regalblecheinsatz und/oder eine bewegte Plattform realisiert, die/der insbesondere aus Edelstahl oder Kuper oder ähnlichem bestehen kann oder dieses Material aufweist. Eine Lagerplatte dient als Bodenplatte, insbesondere als Zwischenbodenplatte. Die Lagerplatte kann der Inkubatorkammer entnehmbar sein ("Lagerplatteneinsatz") oder kann fest eingesetzt mit dieser verbunden sein. Die Inkubatorkammer kann Halteabschnitte oder einen Haltrahmen zum Halten eines oder mehrerer Lagerplatteneinsätze oder einsetzbarer Instrumente aufweisen. Eine Lagerplatte kann an ihrer Unterseite zum Halten einer Kamera eingerichtet sein, insbesondere eine Halterung für diese Kamera aufweisen. Alternativ oder zusätzlich kann mindestens eine der Innenwände der Inkubatorkammer zum Halten eines oder mehrerer Lagerplatteneinsätze oder einsetzbarer Instrumente eingerichtet sein. Dazu kann eine in die Wand integrierte Haltestruktur vorgesehen sein, insbesondere ein oder mehrere Vorsprünge, Rinnen oder Stege. Eine Lagerplatte vergrößert die verfügbare Lagerfläche in der Inkubatorkammer.

Vorzugsweise sind im Wesentlichen alle Oberflächen oder mindestens eine Oberfläche der mindestens eine Lagerplatte nicht-glänzend bzw. nicht-reflektierend ausgestaltet, insbesondere durch Verwendung einer matten Oberfläche. Die Oberfläche der Inkubatorinnenwand kann durch eine Oberflächenbehandlung mattiert sein. Die Oberflächenbehandlung kann insbesondere Schleifen mit einem Schleifmittel sein, das insbesondere eine bestimmte Körnung aufweisen kann. Die Oberflächenbehandlung kann insbesondere Bestrahlen mit einem Strahlmittel, insbesondere Sand oder Glasperlen insbesondere per Druckluft, sein, das insbesondere eine bestimmte Körnung bzw. einen charakteristischen Partikeldurchmesser aufweisen kann. Dadurch können störende Reflexionen in einer Bildaufnahme verhindert bzw. reduziert werden.

Ein Halterahmen für die mindestens eine Lagerplatte ist ebenfalls vorzugsweise aus einem nicht-korrosiven Material gefertigt, vorzugsweise Edelstahl. Der Halterahmen ist vorzugsweise als stehendes Objekt ausgelegt, indem er mindestens einen Sockelabschnitt aufweist, der auf der Bodenwand der Inkubatorkammer ruht. Er kann sich aber auch an den Seitenwänden der Inkubatorkammer abstützen und/oder an der Deckenwand der Inkubatorkammer aufgehängt sein.

Eine Lagerplatte erstreckt sich vorzugsweise - und insbesondere im Wesentlichen vollständig- über einen horizontalen Querschnitt der Inkubatorkammer.

Vorzugsweise weist ein Inkubator mindestens zwei Lagerplatten auf, die übereinander angeordnet sind. Der Volumenbereich zwischen zwei Lagerplatten, bzw. zwischen einer Bodenwand der Inkubatorkammer und einer untersten Lagerplatte oder zwischen einer Deckenwand der Inkubatorkammer und einer obersten Lagerplatte kann als Lagerkompartiment bezeichnet werden. Ein Lagerkompartiment kann insgesamt als Lagerbereich aufgefasst werden. Die zum Lagern geeignete Oberfläche einer Lagerplatte kann als Lagerbereich aufgefasst werden. Die Höhe eines Lagerkompartiments ist vorzugsweise so bemessen, dass ein Objekt einer bestimmten Maximalhöhe (gemessen senkrecht zur planaren Oberfläche einer Lagerplatte) bzw. ein Objektstapel von Objekten einer bestimmten Maximalhöhe des Stapels auf der Lagerplatte abstellbar ist. Die Maximalhöhe kann insbesondere im Wesentlichen dem Abstand zweier Lagerplatten entsprechen.

Der Abstand zwischen zwei Lagerplatten bzw. die Maximalhöhe beträgt insbesondere zwischen 5 cm und 50 cm, vorzugsweise zwischen 10 cm und 30 cm, vorzugsweise zwischen 10 cm und 20 cm, vorzugsweise zwischen 12 cm und 18 cm.

Der Inkubator kann ein Gehäuse aufweisen, dass die Inkubatorkammer teilweise oder vollständig umgibt. Das Gehäuse kann im Wesentlichen quaderförmig ausgebildet sein, und kann insbesondere derart ausgebildet sein, dass der Inkubator stapelbar ist.

Vorzugsweise weist der Inkubator eine Behandlungseinrichtung zur Behandlung des mindestens einen Objekts, insbesondere Zellkulturbehälters auf. Der Begriff "Behandlung" bedeutet insbesondere, dass ein Objekt, insbesondere eine Zellkultur oder ein Zellkulturbehältnis bewegt, und/oder transportiert und/oder untersucht und/oder verändert wird, insbesondere physikalisch, chemisch, biochemisch oder auf andere Weise verändert wird.

Eine Behandlungseinrichtung kann eine Bewegungseinrichtung sein, mittels der das Zellmedium in mindestens einem Zellkulturbehälter in Bewegung gehalten wird, vorzugsweise über ein Bewegungsprogramm, das vom Steuerprogramm gesteuert wird. Eine Bewegungseinrichtung kann eine Schüttel- oder Schwenkeinrichtung sein. Eine Bewegungseinrichtung weist vorzugsweise eine Trägereinrichtung auf, insbesondere eine Platte, auf der ein oder mehrere Zellkulturbehälter abgestellt und/oder fixiert werden. Eine Bewegungseinrichtung weist vorzugsweise eine Antriebseinrichtung auf, insbesondere im Fall einer Schütteleinrichtung beispielsweise einen Oszillatorantrieb, insbesondere in Kombination mit einem Exzenter, mittels dem das gewünschte Bewegungsprogramm umgesetzt wird. Die Gestaltung des Bewegungsprogramms kann vom Wachstumsstadium der Zellen einer Zellkultur abhängen und kann von der Zellart, insbesondere einer Zelllinie abhängen. Die Gestaltung und/oder Steuerung der Behandlung, insbesondere des Bewegungsprogramms, kann von den Zellüberwachungsdaten abhängen. Die Eine Behandlungseinrichtung kann eine Schwenkeinrichtung sein, mittels der mindestens ein Zellkulturbehälter geschwenkt wird. Die Bestandteile der Schwenkeinrichtung können denen der Schütteleinrichtung entsprechen, sind aber für eine Schwenkbewegung eingerichtet.

Eine Behandlungseinrichtung kann auch eine Transporteinrichtung sein, mittels der mindestens ein Zellkulturbehälter in der Inkubatorkammer transportierbar ist. Die Transporteinrichtung kann eine Lifteinrichtung sein, aufweisend eine Trägereinrichtung, auf der der mindestens eine Zellkulturbehälter platzierbar ist. Die Lifteinrichtung weist vorzugsweise einen Bewegungsmechanismus und/oder einen elektrisch ansteuerbaren Antriebsmechanismus zum Antreiben des Bewegungsmechanismus auf. Die Transporteinrichtung kann ferner einen beweglichen und elektrisch ansteuerbaren Greifarm zum Greifen und Halten mindestens eines Zellkulturbehälters sein. Die Transporteinrichtung kann ein Förderband zum Bewegen des mindestens einen darauf platzierten Zellkulturbehälters aufweisen. Durch den Transport kann der mindestens eine Zellkulturbehälter in der Inkubatorkammer bewegt werden, insbesondere zu einer Bearbeitungsposition in einer Bearbeitungsstation in der Inkubatorkammer, und von dieser Bearbeitungsposition weg. Die Steuereinrichtung kann dazu eingerichtet sein, die Transporteinrichtung in Abhängigkeit von Zellüberwachungsdaten zu steuern.

Die Bearbeitungsstation kann mindestens eine Messeinrichtung aufweisen, um den mindestens einen Wachstumsparameter zu messen, der das Wachstum der Zellen dieser Zellkultur charakterisiert. Wenn der mindestens eine Zellkulturbehälter mittels Transporteinrichtung im Inkubator bewegbar ist, können die Zellkulturen mehrerer Zellkulturbehälter mit einer einzigen oder mit wenigen Messeinrichtungen nacheinander gemessen werden. Es können aber auch mehrere oder eine Vielzahl von Messeinrichtungen vorgesehen sein, um parallel das Wachstum mehrerer Zellkulturen zu beobachten.

Die Messeinrichtung kann zudem an einer Transporteinrichtung befestigbar sein. Die Messeinrichtung kann an einem Positioniermechanismus befestigbar sein oder befestigt sein, mittels dem die Messeinrichtung in der Inkubatorkammer bewegbar und positionierbar ist. Der Positioniermechanismus kann einen beweglichen Roboterarm beinhalten und ist vorzugsweise elektrisch steuerbar, insbesondere durch ein Steuerprogramm der Steuereinrichtung. Auf diese Weise kann das Wachstum der Zellen mehrerer Zellkulturbehälter nacheinander mit einer oder mit wenigen Messeinrichtungen nacheinander gemessen werden. Der Positioniermechanismus kann als in die Inkubatorkammer einsetzbares Bauteil ausgebildet sein. Die Energiezufuhr dieses Bauteils kann über eine Kabelverbindung mit dem Inkubator erfolgen, vorzugsweise über ein durch eine Wandöffnung, z.B. einen Port, oder über eine solche Kabelverbindung zu einer externen Spannungsquelle. Die Steuereinrichtung kann dazu eingerichtet sein, den Positioniermechanismus in Abhängigkeit von Zellüberwachungsdaten zu steuern.

Als Behandlungseinrichtung lässt sich auch die Temperiereinrichtung der Inkubatorkammer verstehen, mit der die Atmosphäre im Inneren der Inkubatorkammer auf den gewünschten Wert, insbesondere 37°C geregelt wird. Der Begriff Temperieren bezieht sich auf das Erhöhen und Senken der Atmosphärentemperatur durch Heizen und Kühlen. Vorzugsweise wird die Temperatur im Inneren über eine Temperaturänderung der Wände des Inkubators eingestellt. Temperatursensoren der entsprechenden Temperaturregeleinrichtung sind an wenigstens einer Position im Inneren und / oder außerhalb der Inkubatorkammer, insbesondere an einer Wand der Inkubatorkammer verteilt.

Der Inkubator weist vorzugsweise eine Benutzerschnittstelleneinrichtung auf, über die der Benutzer Daten an die Datenverarbeitungseinrichtung bzw. die Steuereinrichtung eingeben kann, und/oder über die Informationen an den Benutzer ausgegeben werden können. Vorzugsweise ist der Inkubator bzw. diese Benutzerschnittstelleneinrichtung dazu eingerichtet, dass der Benutzer mindestens einen Betriebsparameter zum Betreiben des Inkubators oder des Objektverfolgungssystems an dieser Benutzerschnittstelleneinrichtung eingeben kann bzw. Informationen von dieser erhalten kann. Auf diese Weise kann eine einzige Benutzerschnittstelleneinrichtung vom Benutzer dazu verwendet werden, um den Inkubator und auch die mindestens Objektverfolgungssystems zu beeinflussen, oder zu steuern, oder von diesen Informationen zu erhalten. Insbesondere kann das Objektverfolgungssystem dazu eingerichtet sein, dem Benutzer auf eine mittels der Benutzerschnittstelleneinrichtung des Inkubators erfolgte Abfrage des Benutzers Positionsdaten oder freien Lagerplatz anzuzeigen, oder aus Positionsdaten abgeleitete Informationen, (z.B. Identität des Benutzers, der die Positionsänderung herbeigeführt hat), insbesondere auch statistische Informationen, wie beispielsweise Häufigkeit und Zeitpunkt der Positionsveränderung eines Objektes (einer Probe) und / oder -z.B. prozentual- verfügbarer freier Lagerplatz und/oder mindestens ein optisches Abbild des mindestens einen Objektes anzuzeigen. Für den Benutzer ist dies vorteilhaft, da er aufgrund dieser Informationen wesentliche Informationen erhält, die ihm zum einen eine genauere Versuchsplanung erlauben - bevor er einen Versuch durchführt weiß er, dass Stellfläche verfügbar ist; zum anderen beeinflusst die Positionsänderung von Proben, insbesondere in den ersten Stunden nach der Aussaat von adhärenten Zellen, negativ deren Anhaftung; es bildet sich dann kein gleichmäßiger Zellrasen aus. Die erfindungsgemäße Bereitstellung der Informationen zu Positionsänderungen bzw. deren Häufigkeit erlaubt es dem Benutzer Ursachen für ein ungleichmäßiges Zellwachstum zu ermitteln und diese so bei zukünftigen Versuchen zu berücksichtigen.

Eine gerätegesteuerte Behandlung des Inkubators ist vorzugsweise eine programmgesteuerte Behandlung, also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mittels der digitalen Datenverarbeitungseinrichtung, die insbesondere Bestandteil der Steuereinrichtung ist. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich. Eine gerätegesteuerte Behandlung des Inkubators kann insbesondere in Abhängigkeit von der Endposition und/oder den benutzerbezogenen ID-Positionsdaten erfolgen.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Unter einem Programm wird insbesondere ein Computerprogramm verstanden. Ein Programm ist eine Folge von Anweisungen, insbesondere bestehend aus Deklarationen und Instruktionen, um auf einer digitalen Datenverarbeitungseinrichtung eine bestimmte Funktionalität, Aufgaben- oder Problemstellung bearbeiten und/oder lösen zu können. Ein Programm liegt in der Regel als Software vor, die mit einer Datenverarbeitungseinrichtung verwendet wird. Das Programm kann insbesondere als Firmware vorliegen, im Fall der vorliegenden Erfindung insbesondere als Firmware der Steuereinrichtung des Inkubators oder des Systems. Das Programm liegt meist auf einem Datenträger als ausführbare Programmdatei, häufig im sogenannten Maschinencode vor, die zur Ausführung in den Arbeitsspeicher des Rechners der Datenverarbeitungseinrichtung geladen wird. Das Programm wird als Abfolge von Maschinen-, d. h. Prozessorbefehlen von dem/den Prozessoren des Computers verarbeitet und damit ausgeführt. Unter ,Computerprogramm' wird insbesondere auch der Quelltext des Programms verstanden, aus dem im Verlauf der Steuerung des Laborgerätes der ausführbare Code entstehen kann.

Eine Benutzerschnittstelleneinrichtung kann Bestandteil eines Inkubators sein, oder ein Modul. Eine Benutzerschnittstelleneinrichtung weist jeweils vorzugsweise auf: eine Steuereinrichtung für die Benutzerschnittstelleneinrichtung; eine Kommunikationseinrichtung zur Herstellung einer Datenverbindung mit einem Laborgerät, insbesondere einem Inkubator, über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer, insbesondere ein berührungsempfindliches Display. Dabei ist die Steuereinrichtung der Benutzerschnittstelleneinrichtung vorzugsweise dazu eingerichtet, über die Datenverbindung Daten mit der Steuereinrichtung des Inkubators auszutauschen.

Das in den Inkubator eingestellte und mittels Objektverfolgung verfolgte Objekt kann insbesondere ein Zellkulturbehälter sein, oder ein anderer Laborprobenhalter, z.B. ein Objektträger, oder kann ein anderer Laborgegenstand sein, z.B. ein Gerät, insbesondere ein Wippschüttler, ein Shaker, ein pH-Meter.

Ein Objekt ist insbesondere ein Zellkulturbehälter. Ein Zellkulturbehälter ist insbesondere transparent. Er ist insbesondere aus Glas oder Kunststoff gefertigt, insbesondere PE oder PS gefertigt und weist insbesondere eine planare Bodenplatte auf, welche die Wachstumsfläche der Zellen bildet. Diese kann eine Oberflächenbehandlung zur Förderung der Adhärenz von Zellen aufweisen. Der Zellkulturbehälter kann mit einer PE-Kappe oder Gasaustauschkappe, insbesondere einem Deckel mit optional enthaltenem Filter, verschließbar sein bzw. versehen sein. Der Zellkulturbehälter ist insbesondere stapelbar. Geeignet ist insbesondere eine Eppendorf Zellkulturflasche. Das Objekt kann ein Stapel von Zellkulturbehältern sein, insbesondere ein Stapel von Petrischalen oder von Zellkulturflaschen.

Die Kamera des Objektverfolgungssystems kann eine Weitwinkeloptik, insbesondere eine Fischaugenoptik aufweisen, deren Bildwinkel in der Bilddiagonalen zwischen 160° und 180° betragen kann. Vorzugsweise ist eine Kamera -oder sind mehrere Kameras- an der Unterseite eines Regalblecheinsatzes in der Inkubatorkammer montiert, oder an einer Unterseite der oberen Innenwand der Inkubatorkammer, vorzugsweise jeweils vertikal oberhalb des geometrischen Zentrum etc. s des Lagerbereichs, den die Kamera überwacht. Eine oder mehrere Kameras können aber auch an inneren Seitenwänden der Inkubatorkammer angeordnet / befestigt sein. "Unten" bezeichnet die Richtung der Gravitation, "Oben" die entgegengesetzte Richtung. Im bestimmungsgemäßen Gebrauch sind Inkubatoren so angeordnet, dass die Oberseiten der planaren Regaleinsätze horizontal liegen. Die Kamera des Inkubators ist insbesondere geeignet, um in der jeweiligen Inkubatoratmosphäre über einen Zeitraum von mehreren Monaten oder Jahren zuverlässig zu arbeiten, bzw. um während der unter Standardbedingungen (Raumtemperatur) gemessenen Lebensdauer zuverlässig zu arbeiten. Nicht jede Kamera ist geeignet, um in einer Inkubatoratmosphäre zu funktionieren. Eine mögliche, kommerziell erhältliche Kamera ist die 5MP Weitwinkel Camera für Raspberry Pi, www.jov-it.net, erhältlich bei Conrad Electronic SE, Deutschland, und/oder eine andere Kamera in Kombination mit einer Weitwinkellinse, z.B. kommerziell erhältlich die "Industrial lens HAL 250 2.3", Entaniya Co.,Ltd., Japan. Alternativ kann eine Hülleinrichtung für die mindestens eine Kamera vorgesehen sein, um diese gegenüber der Inkubatoratmosphäre abzuschirmen bzw. zu isolieren, wobei diese Hülleinrichtung insbesondere transparente Bereiche oder ein transparentes Fenster aufweist bzw. transparent ist, um die Bildaufnahmen durch die transparenten Bereich zu ermöglichen.

Vorzugsweise ist die Datenverarbeitungseinrichtung dazu programmiert, aus einem oder mehreren Standbildern, und/oder dem Startbild , und/oder dem Endbild , und/oder aus den Videodaten (zeitabhängige) Änderungen des Erscheinungsbildes (oder das Erscheinungsbild) der Objekte, insbesondere zwischen längeren Zeitabständen von Minuten, Stunden oder Tagen, zu erfassen. Auf diese Weise können Farbänderungen des Zellkulturmediums bzw. Farben in einem Zellkulturbehälter oder Strukturen, z.B. Tropfen, an einer Zellkulturbehälterwand ermittelt werden. Solche Farben, Farbänderungen oder Strukturen können auf Probleme der jeweiligen Zellkultur hinweisen, z.B. auf einen Nährstoffmangel, pH-Wert Veränderung und/ oder auf Schimmel, und/oder auf andere Kontaminationen. Vorzugsweise ist die Datenverarbeitungseinrichtung dazu programmiert, abhängig von der Detektion des Erscheinungsbildes eines Zellkulturbehälters bzw. dieser Änderungen des Erscheinungsbildes des Zellkulturbehälters eine Information über eine Benutzerschnittstelle an den Benutzer oder Betriebspersonal auszugeben und/oder die Daten über diese Detektion (insbesondere: was wurde wann detektiert) in einem Datenspeicher zu speichern.

Vorzugsweise ist die Datenverarbeitungseinrichtung dazu programmiert, aus einem oder mehreren Standbildern, und/oder dem Startbild, und/oder dem Endbild , und/oder aus den Videodaten (zeitabhängige) Änderungen des Erscheinungsbildes der Objekte, insbesondere zwischen längeren Zeitabständen von Minuten, Stunden oder Tagen zu ermitteln.

Die Erfindung betrifft insbesondere auch ein System zur Inkubation lebender Zellkulturen, aufweisend
einen Inkubator zur Inkubation lebender Zellkulturen, der aufweist:
   eine Inkubatorkammer zur Aufnahme von Objekten, insbesondere Zellkulturbehältern, in mindestens einem Lagerbereich der Inkubatorkammer, die eine verschließbare Kammeröffnung zur Auf- und Entnahme der Objekte aufweist,
   eine Datenverarbeitungseinrichtung und einen Datenspeicher,
ein zum Nachrüsten des Inkubators eingerichtetes bildverarbeitendes Objektverfolgungssystem zur Verfolgung von Positionsänderungen mindestens eines in die Inkubatorkammer eingebrachten Objektes ausgehend von dessen Startposition in einem Startbild eines Lagerbereichs bis zu dessen Endposition in einem Endbild des Lagerbereichs, wobei das Objektverfolgungssystem
mindestens eine Datenverarbeitungseinrichtung, einen Datenspeicher und mindestens eine Kamera aufweist, die dazu eingerichtet ist, einen Innenraum der Inkubatorkammer zu überwachen,
wobei die Datenverarbeitungseinrichtung dazu programmiert ist,
dem in den Innenraum eingebrachten mindestens einen Objekt Identifikationsdaten zuzuordnen,
die Startposition des mindestens einen Objektes aus dem Startbild des Lagerbereichs und die Positionsänderungen des mindestens einen Objektes durch Auswertung der Videodaten zu ermitteln,
aus den Positionsänderungen die Endposition des mindestens einen Objektes im Endbild des Lagerbereichs zu ermitteln, und
die Endposition des mindestens einen Objektes im Lagerbereich in Abhängigkeit von den Identifikationsdaten als ID-Positionsdaten in dem Datenspeicher abrufbar zu speichern.

Das vorstehend genannte System beruht somit auf einem Inkubator, der mit einem nachrüstbaren Objektverfolgungssystem nachrüstbar ist, so wie dieses im Anspruch 1 fester Bestandteil des Inkubators ist, wobei das nachrüstbare Objektverfolgungssystem entsprechend kompatibel sein muss mit dem so bezeichneten "kompatiblen Inkubator".

Vorzugsweise ist eine Steuereinrichtung des erfindungsgemäßen Inkubators oder des kompatiblen Inkubators, die insbesondere auch die atmosphärischen Parameter in der Inkubatorkammer steuern kann (Temperatur, Gaspartialdruck CO₂, H₂O etc.) bzw. deren Datenverarbeitungseinrichtung dazu eingerichtet bzw. programmiert, in Abhängigkeit von Daten aus dem Objektverfolgungssystem, insbesondere von Positionsdaten bzw. von der Endposition mindestens einen Objektes, mindestens einen Betriebsparameter des Inkubators zu bestimmen, insbesondere einen Parameter, der die Anzeige von Informationen auf einem Bildschirm des Inkubators steuert oder einen Parameter, der auf dem Bildschirm des Inkubators angezeigt wird. Insbesondere können Positionsdaten bzw. die Endposition mindestens einen Objektes auf dem Bildschirm wiedergegeben werden.

Vorzugsweise weist das System zur Inkubation lebender Zellkulturen auf: ein getrennt vom Inkubator mit diesem in Datenaustauschverbindung stehendes Externgerät, insbesondere eine -insbesondere mobile- Benutzeridentifikationseinrichtung und insbesondere eine Datenaustauscheinrichtung, mittels der die Datenverarbeitungseinrichtung mit dem Externgerät Daten austauschen kann, insbesondere anhand der Benutzeridentifikationseinrichtung Benutzeridentifikationsdaten ermitteln kann.

Die Erfindung bezieht sich auch auf ein Verfahren zur Verfolgung der Objektpositionierungen in einem Inkubator, welcher der Inkubation von lebenden Zellkulturen in einer Inkubatorkammer des Inkubators dient, aufweisend die computergesteuerten Schritte:
* Überwachen der Inkubatorkammer mittels mindestens einer Kamera des Inkubators, die zur Aufnahme mindestens eines Lagerbereichs im Innenraums der Inkubatorkammer angeordnet ist, in den das mindestens eine Objekt eingebracht wird;
* Zuordnen von Identifikationsdaten zu dem mindestens einen Objekt, das in einem mittels der mindestens einen Kamera aufgenommenen Startbild des Lagerbereichs beim Einbringen in den Innenraum erfasst wird;
* Erfassen von Positionsänderungen des mindestens einen Objektes durch Auswertung von mittels der Kamera gewonnenen Videodaten; insbesondere wird die zeitgleiche Bewegung mehrerer Objekte, insbesondere Bestandsobjekte ermöglicht bzw. ausgeführt;
* Ermitteln der Endposition des mindestens einen Objektes in einem Endbild des Lagerbereichs aus den Positionsänderungen;
* Speichern der Endposition des mindestens einen Objektes im Lagerbereich in Abhängigkeit von den Identifikationsdaten als ID-Positionsdaten, in dem Datenspeicher, insbesondere abrufbares Speichern, also Speichern und Bereitstellen der jeweiligen Daten, um den Zugriff einer Datenverarbeitungseinrichtung und das Auslesen der Daten zu ermöglichen.

Bei dem Verfahren wird vorzugsweise genau ein Objekt (kann auch Stapel oder Gruppe von Objekten sein) in den Innenraum eingebracht und deren Endposition ermittelt. Es ist auch möglich und bevorzugt, dass dabei registriert wird, dass während des Einstellens des einen Objektes mindestens ein Bestandsobjekt bewegt wird, indem es z.B. mittels des einen eingebrachten Objektes geschoben wird, und somit die Endposition des mindestens einen Bestandsobjektes aus dessen per Bildverarbeitung ermittelten Positionsänderungen ermittelt wird. Es ist auch möglich und bevorzugt, dass bei dem Verfahren mehrere Objekte in den Innenraum eingeführt werden, gleichzeitig und/oder nacheinander, und dass diesen Objekten aus einem Startbild jeweils eine Identifikationsnummer zugeordnet wird, deren Positionsänderungen aus Videodaten ermittelt werden und daraus jeweils deren Endposition in einem Endbild bestimmt wird.

Vorzugsweise weist das Verfahren den Schritt auf: Einlesen von einen den Benutzer des Inkubators, der das mindestens eine Objekt in die Inkubatorkammer einbringt, identifizierenden Benutzeridentifikationsdaten mittels einer Benutzeridentifikationseinrichtung und insbesondere Speichern der Benutzeridentifikationsdaten in einem Datenspeicher des Inkubators.

Vorzugsweise weist das Verfahren den Schritt auf: Speichern der Positionsdaten des mindestens einen Objektes in Abhängigkeit von den Benutzeridentifikationsdaten als benutzerbezogene Objektpositionsdaten.

Die Erfindung bezieht sich auch auf ein, insbesondere zum Nachrüsten eines Inkubators eingerichtetes, bildverarbeitendes Objektverfolgungssystem zur Verfolgung von Positionsänderungen mindestens eines in die Inkubatorkammer eingebrachten Objektes ausgehend von dessen Startposition in einem Startbild eines Lagerbereichs bis zu dessen Endposition in einem Endbild des Lagerbereichs, wobei das Objektverfolgungssystem
mindestens eine Datenverarbeitungseinrichtung, einen Datenspeicher und mindestens eine Kamera aufweist, die dazu eingerichtet ist, einen Innenraum der Inkubatorkammer zu überwachen,
wobei die Datenverarbeitungseinrichtung dazu programmiert ist,
dem in den Innenraum eingebrachten mindestens einen Objekt Identifikationsdaten zuzuordnen,
die Startposition des mindestens einen Objektes aus dem Startbild des Lagerbereichs und die Positionsänderungen des mindestens einen Objektes durch Auswertung der Videodaten zu ermitteln,
aus den Positionsänderungen die Endposition des mindestens einen Objektes im Endbild des Lagerbereichs zu ermitteln, und
die Endposition des mindestens einen Objektes im Lagerbereich in Abhängigkeit von den Identifikationsdaten als ID-Positionsdaten in dem Datenspeicher zu speichern.

Weitere bevorzugte Ausgestaltungen des erfindungsgemäßen Verfahrens lassen sich aus der Beschreibung des erfindungsgemäßen Inkubators und dessen bevorzugten Ausgestaltungen entnehmen. Ferner ergeben sich weitere Ausgestaltungsoptionen der Erfindung aus den Ausführungsbeispielen in den Figuren. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1 zeigt einen erfindungsgemäßen Inkubator gemäß einem Ausführungsbeispiel in perspektivischer Ansicht.
Fig. 2 zeigt den Inkubator aus Fig. 1 in einer Frontansicht.
Fig. 3 zeigt den Inkubator aus Fig. 1 in einer Frontansicht mit graphischer Darstellung der Belegung der Inkubatorkammer mit Objekten, die benutzerbezogen farbcodiert hervorgehoben sind.
Fig. 4a zeigt ein Smartphone mit Kamera und Display 63 als Externgerät, das Bestandteil eines Systems 400 umfassend den Inkubator 1 aus Fig. 3 und das Smartphone 69 sein kann.
Fig. 4b zeigt eine Legende der im Bildschirm der Fig. 3 verwendeten Farbcodierung zur Hervorhebung benutzerbezogener Objekte.
Fig. 5a zeigt in einer schematischen Seitenansicht ein Objektverfolgungssystem als Bestandteil des Inkubators aus den Fig. 1 bis 4b in einem Beispiel einer Kammer mit einer einzigen überwachten Lagerplatte.
Fig. 5b zeigt in einer schematischen Seitenansicht ein Objektverfolgungssystem als Bestandteil des Inkubators aus den Fig. 1 bis 4b in einem Beispiel einer Kammer mit mehreren überwachten Lagerplatten.
Fig. 5c zeigt eine perspektivische Ansicht eines Lagerbereichs, der vom Objektverfolgungssystem der Fig. 5a und 5b überwacht wird, sowie die auf ein Koordinatensystem bezogene Startposition P1, Positionsänderungen dP und Endposition P2 eines verfolgten Objektes.
Fig. 5d zeigt ein von der Weitwinkel-Fischaugenkamera des in Fig. 5a und 5b verwendeten Objektverfolgungssystems erfasstes, aufgrund der Optik verzerrt erscheinendes digitales Bild.
Fig. 5e zeigt das Bild der Fig. 5d, das vom Objektverfolgungssystem durch Begradigungsalgorithmen entzerrt wurde.
Fig. 5f zeigt ein von der Weitwinkel-Fischaugenkamera des in Fig. 5a und 5b verwendeten Objektverfolgungssystems erfasstes Standbild zur Ausgabe auf einem Bildschirm des Inkubators, bei dem die Bounding Boxes des Objektverfolgungssystems, Identifikationsnummern und eine den Benutzer/Eigentümer identifizierende Farbkodierung gezeigt sind.
Fig. 5g zeigt einen möglichen Bildschirminhalt, der zur Erläuterung der in Fig. 5f gezeigten Bildschirmseite auf einem Bildschirm des Inkubators angezeigt werden kann.
Fig 6 zeigt in einer schematischen Aufsicht einen Lagerbereich des Inkubators aus Fig. 1 bis 5, inklusive Objekten, der in einem Bilderfassungsausschnitt einer Kamera des Objektverfolgungssystems angeordnet ist.
Fig. 7 zeigt anhand des Ausschnitts aus Fig. 6 das Verdrängen eines Bestandsobjektes durch ein neu in den Inkubator hineinbewegtes Objekt.
Fig. 8a bis 8e zeigen jeweils eine andere Bewegungsphase der Objekte im Ausschnitt der Fig. 6, mit Definition der Bounding Box der bewegten Objekte durch Algorithmen des Objektverfolgungssystems. Fig. 8a zeigt das Startbild, Fig. 8f zeigt das Endbild.
Fig. 9 zeigt schematisch den Ablauf eines beispielhaften erfindungsgemäßen Verfahrens.

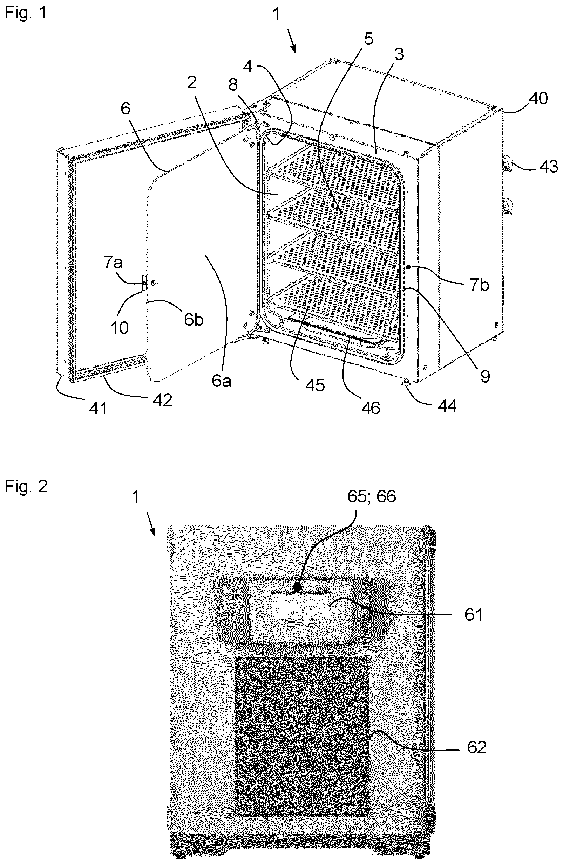

Fig. 1a zeigt einen Inkubator 1 zum Lagern von Laborproben, genauer gesagt einen CO2-Inkubator zur Lagerung von lebenden Zellkulturen in einer definierten Atmosphäre bei einer geregelten Temperatur, z.B. 37° C. Zu diesem Zweck ist der Kammerinnenraum 5 des Inkubators thermisch isoliert und gasdicht gegenüber der Umgebung abschließbar, die Gaszusammensetzung im Innenraum ist ebenfalls geregelt und kann über Gasanschlüsse 43 geändert werden. Das Kammergehäuse 2 des Inkubators steht auf Sockeln 44, kapselt den Innenraum 5 ein und mündet in der Frontseite 3 des Inkubators. Die Frontseite weist die Kammeröffnung 4 auf, durch die der Kammerinnenraum 5 zugänglich ist. Eine durchsichtige innere Kammertüre 6 dient dem Schließen der Kammeröffnung in einer Schließposition der Kammertüre. Beim Inkubator 1 ist das Kammergehäuse 2 innerhalb des Innenraums eines Außengehäuses 40 platziert, so dass das Kammergehäuse 2 und das Außengehäuse 40 voneinander beabstandet und gegeneinander thermisch isoliert sind. Im Kammerinnenraum sind Regalblecheinsätze 45 und eine Luftbefeuchterwanne 46 zu sehen. Die Frontseite 3 des Kammergehäuses und die Frontseite des Außengehäuses fallen vorliegend zusammen.

Die äußere Inkubatortüre 41 und die Kammertüre 6 sind in einer geöffneten Position gezeigt. Die Außentüre 41 ist über Scharniere an der Außenkante des Außengehäuses schwenkbar gelagert und weist eine umlaufende Silikondichtung 42 auf.

Wenn die Außentüre 41 geöffnet wurde, ist die innere Kammertüre 6 des Inkubators zunächst noch geschlossen. Dazu dient die Verschlusseinrichtung (10, 7a, 7b). Bei geschlossener Kammertüre 6 kann der Benutzer zunächst den Innenraum 5 durch die transparente Türwand sichten, bevor er die Tür öffnet und eine Laborprobe einsetzt oder entnimmt. Dennoch stellt das Öffnen der äußeren Inkubatortüre 41 bereits eine Störung dar, die die Inkubatoratmosphäre potentiell schädigen kann.

Der Inkubator weist eine in die Türe 41 verbaute und nach vorne ausgerichtete Außenkamera 65 auf, deren Bilder von der geeignet programmierten Datenverarbeitungseinrichtung des Inkubators auswertbar sind, insbesondere um einen Benutzer mittels Gesichtserkennung zu identifizieren, wodurch die mit der Datenverarbeitungseinrichtung verbundene Außenkamera 65 als Benutzeridentifikationseinrichtung 66 dient. Letzteres kann auch über die Kamera des Smartphones 69 erfolgen.

Um die gelagerten Laborproben zu schützen, ist es beim Inkubator wirksam, die Zeit zu minimieren, während der der Innenraum des Inkubators gegenüber der Umgebung exponiert ist (Öffnungszeitintervalle). Der vorliegenden Erfindung liegt die Beobachtung zugrunde, dass die Öffnungszeitintervalle durch ein Objektverfolgungssystem reduziert werden können. Der Inkubator 1 weist ein Objektverfolgungssystem auf (nicht gezeigt in Fig. 1, 2).

Die Außenseite der äußeren Inkubatortüre weist, wie in Fig. 2 gezeigt ist, einen ersten Bildschirm auf, einen Touchscreen 61, über den Betriebsparameter des Inkubators 1 angezeigt werden, z.B. die Temperatur der Inkubatoratmosphäre oder einen Gaspartialdruck im Innenraum 5.

Die Außenseite der äußeren Inkubatortüre 41 weist einen zweiten Bildschirm 62 auf, der ein Touchscreen sein kann. Die Datenverarbeitungseinrichtung (nicht gezeigt) des Inkubators 1 ist dazu programmiert, im Bildschirm 62 die Belegung des Innenraums des Inkubators anzuzeigen. Der Bildschirm 62 dient als "digitales Fenster", das dem Benutzer den (virtuellen) Blick ins Inkubatorinnere ermöglicht. Dabei kann die graphische Wiedergabe des Innenraums des Inkubators und dessen Belegung mit Bestandsobjekten so programmiert sein, dass bestimmte Bestandsobjekte abhängig von bestimmten Kriterien bzw. Bedingungsparametern graphisch hervorgehoben sind.

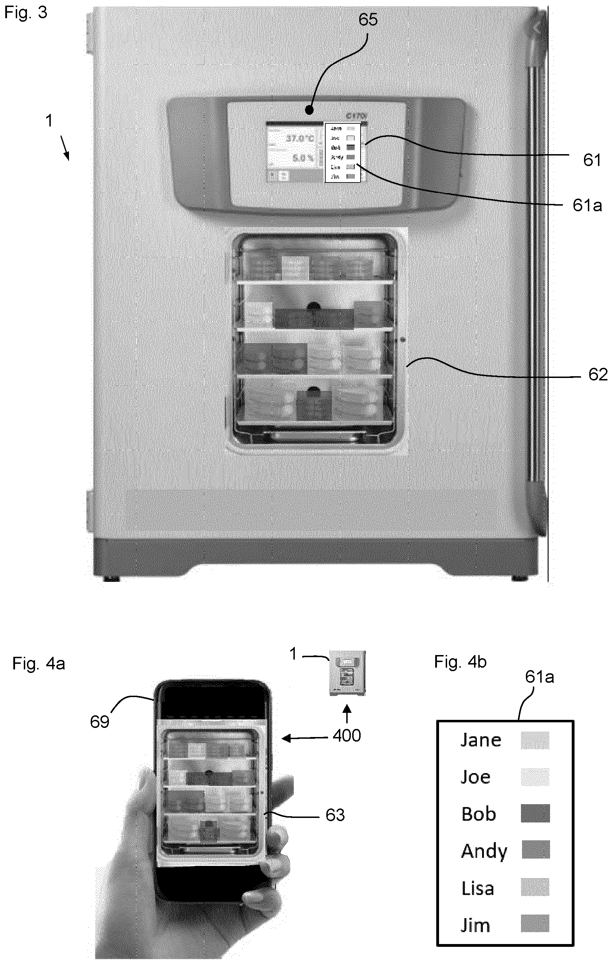

Zu Fig. 3: Die Datenverarbeitungseinrichtung des Inkubators 1 ist dazu programmiert, in Abhängigkeit von mindestens einem Bedingungsparameter, der hier von Benutzeridentifikationsdaten abhängt, ein Objekt oder mehrere Objekte im Display 62 anzuzeigen, und zwar gemäß deren jeweiliger Endposition im Innenraum des Inkubators, die mittels des Objektverfolgungssystems ermittelt wurde. Es sind jeweils die mit bestimmten Benutzeridentifikationsdaten, die mit einen bestimmten Benutzer kennzeichnen, assoziierten Bestandsobjekte mit einer bestimmten nutzerabhängigen Farbe farblich hervorgehoben Die Legende 61a zu dieser Art von Farbcodierung ist dem Benutzer hier über das obere Display 61 in dessen Unter-Bereich 61a angezeigt. Die Legende 61a ist in Fig. 4b größer gezeigt: den Benutzerkennungen "Jane", Joe" etc. sind die entsprechenden, im Display 62, 63 verwendeten Hervorhebungsfarben zugeordnet.

In Fig. 4 ist gezeigt, dass das Ausgabedisplay 61 und/oder 62 auch -alternativ oder zusätzlich- Bestandteil(e) eines Externgeräts sein kann/können, hier ein Smartphone 69, das in einer Datenaustauschverbindung mit dem Inkubator steht und das Display 63 aufweist, das hier als Bestandteil eines Inkubatorsystems fungiert.

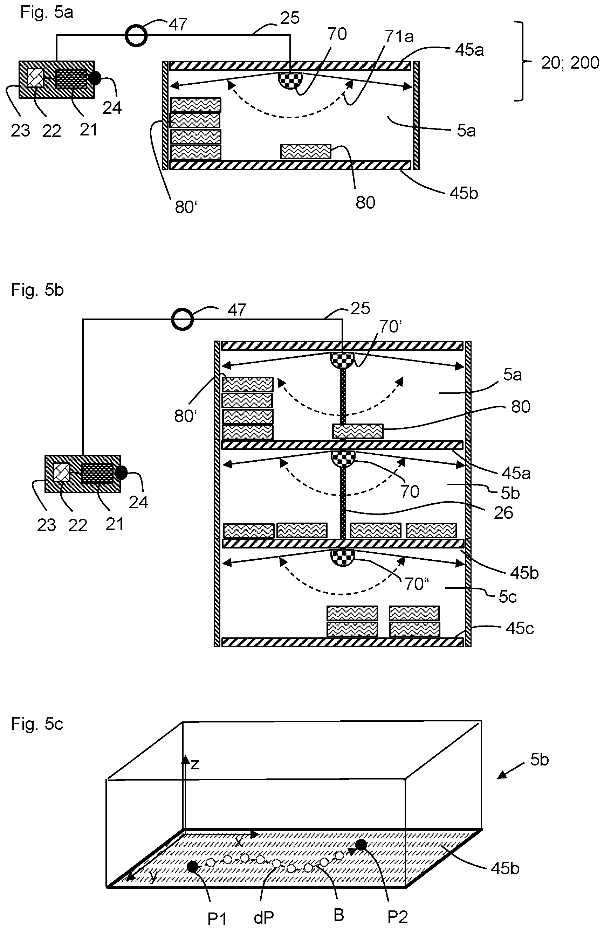

Fig. 5a zeigt in einer schematischen Frontansicht Regaleinsätze 45a und 45b des Inkubators 1, die übereinander angeordnet sind. Der vertikale Abstand solcher Regaleinsätze 45 in Inkubatoren ist meist nicht groß und liegt z.B. zwischen 10 und 40 cm, beim Inkubator 1 etwa bei 15 cm. Dadurch müssen entweder mehrere Kameras verwendet werden, um den gesamten Lagerbereich, hier die gesamte Lagerfläche des Regaleinsatzes 45b und den darüber liegenden "Luftraum" bis zum Regaleinsatz 45a zu erfassen. Die Kamera 70 ist eine Weitwinkel-Fischaugen-Kamera mit einem Bildwinkel von ca. 180°.

Fig. 5a zeigt das im Inkubator 1 eingebaute Objektverfolgungssystem 20, das im Falle der Ausführung als Nachrüstsystem auch mit dem Bezugszeichen 200 bezeichnet ist. Das Objektverfolgungssystem 20 beinhaltet die Kamera 70, eine Weitwinkel-Fischaugenkamera, die im Blickwinkel 71a von vorzugsweise 160° bis 170° den unter ihr liegenden Lagerbereich des Regalblecheinsatzes 45b erfasst. Der weite Blickwinkel ermöglicht es, mit einer einzigen Kamera auszukommen, um den gesamten Lagerbereich zu des unten liegenden Regalblecheinsatzes 45b zu erfassen, insbesondere auch den Luftraum, in den die (Bestands-)Objekte 80' und 80 hineinragen, nämlich ein Stapel 80' von Zellkulturbehältern, und ein Zellkulturbehälter 80. Der nominale Sichtwinkel der Weitwinkel-Fischaugenkamera beträgt sogar 200°, es wird aber nur ein Bildbereich ausgewertet, der einem Sichtwinkel entnommen aus dem Bereich von vorzugsweise 160° bis 170° entspricht.

Die Kamera ist vertikal über dem geometrischen Zentrum der Lagerfläche des Regalblecheinsatzes 45b angeordnet. Das Objektverfolgungssystem 20 beinhaltet auch die Steuereinrichtung 23, welche eine Datenverarbeitungseinrichtung 21 und einen Datenspeicher 22 als weitere Bestandteile des Objektverfolgungssystems 20 aufweist. Die Datenverarbeitungseinrichtung 21 bzw. die Steuereinrichtung 23 sind über eine Kabelverbindung 25, die durch den Port 47 in der Inkubatorkammerrückwand in die Inkubatorkammer gelangt, mit der Kamera 70 und den nicht gezeigten weiteren drei Kameras verbunden, die jeweils vorgesehen sind, damit alle Lagerbereiche (alle Oberseiten von Regalblecheinsätzen 45, siehe Fig. 1) überwacht werden. Die Steuereinrichtung 23 weist zudem eine Datenschnittstelle 24 auf, über die eine Datenverbindung zu weiteren Inkubatorgerätebestandteilen ermöglicht wird, z.B. um Daten bzw. Signale an ein Display 61, 62, 63 des Inkubators auszugeben.

Fig. 5b zeigt in einer schematischen Seitenansicht ein Objektverfolgungssystem als Bestandteil des Inkubators aus den Fig. 1 bis 4b in einem Beispiel einer Kammer mit mehreren überwachten Lagerplatten. Die Darstellung ist eine Erweiterung des Prinzips aus Fig. 5a, bei dem die Inkubatorkammer in mehrere Kompartimente 5a, 5b und 5c unterteilt ist, die hier übereinander angeordnet sind und zum Gasaustausch verbunden sind, was über Löcher in den Lagerplatten 45a, 45b, 45c gebildet ist. Der Lagerbereich bzw. die Lagerplatte 45a im Kompartiment 5a wird von der Kamera 70' überwacht, der Lagerbereich bzw. die Lagerplatte 45b im Kompartiment 5b wird von der Kamera 70' überwacht, und der Lagerbereich bzw. die Lagerplatte 45c im Kompartiment 5c wird von der Kamera 70" überwacht, wobei die Kameras 70' und 70" analog zur Kamera 70 in Fig. 5a ausgeführt und angeordnet sind. Alle Kameras sind über ein Verbindungskabelbündel 26 im Inneren der Inkubatorkammer, das in die bereits in Figur 5a gezeigte Kabelverbindung 25 übergeht und durch den Port 47 in der Inkubatorkammerrückwand die Inkubatorkammer verlässt, mit der Steuereinrichtung 23 verbunden, deren Datenverarbeitungseinrichtung 21 zur Überwachung aller Objekte in allen drei Kompartimenten 5a, 5b und 5c eingerichtet ist. Das Objektverfolgungssystem des zu Fig. 5b gehörenden Inkubators umfasst hier drei Kameras 70, 70', 70" die Datenverarbeitungseinrichtung 21 und die Datenspeichereinrichtung 22.

Fig. 5c zeigt eine perspektivische Ansicht eines Kompartiments 5b bzw. Lagerbereichs 45b, der vom Objektverfolgungssystem der Fig. 5a und 5b überwacht wird, sowie die auf ein kartesisches Koordinatensystem (x, y, z) bezogene Startposition P1, Positionsänderungen dP und Endposition P2 eines auf seinem Bewegungspfad B verfolgten Objektes. Der Ursprung des Koordinatensystems kann in einer Ecke des Kompartiments fest lokalisiert sein.

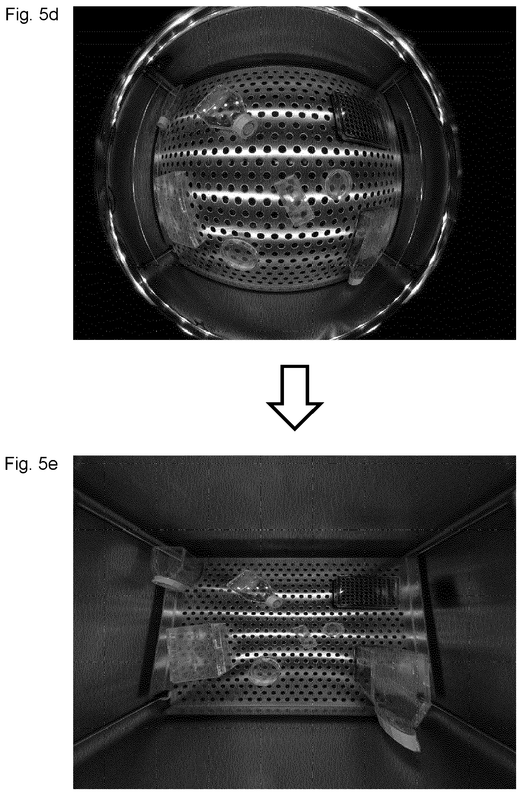

Fig. 5d zeigt ein von der Weitwinkel-Fischaugenkamera des in Fig. 5a und 5b verwendeten Objektverfolgungssystems erfasstes, aufgrund der Optik verzerrt erscheinendes digitales Bild.

Fig. 5e zeigt das Bild der Fig. 5d, das vom Objektverfolgungssystem durch Begradigungsalgorithmen entzerrt wurde.

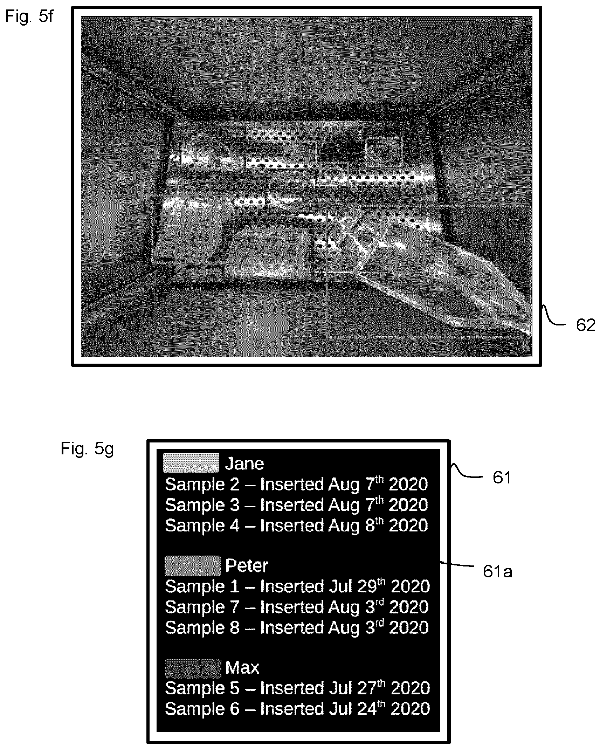

Fig. 5f zeigt ein von der Weitwinkel-Fischaugenkamera des in Fig. 5a und 5b verwendeten Objektverfolgungssystems erfasstes Standbild zur Ausgabe auf einem Bildschirm des Inkubators, bei dem die Bounding Boxes des Objektverfolgungssystems, Identifikationsnummern und eine den Benutzer/Eigentümer identifizierende Farbkodierung gezeigt sind.

Fig. 5g zeigt einen möglichen Bildschirminhalt, der zur Erläuterung der in Fig. 5f gezeigten Bildschirmseite auf einem Bildschirm des Inkubators angezeigt werden kann. Neben der Identifizierung der Objekte durch Identifikationsnummern ist auch eine den Benutzer/Eigentümer identifizierende Farbkodierung gezeigt, sowie die vom Objektverfolgungssystem registrierten Zeitpunkte des Einstellens der Objekte in die Inkubatorkammer.

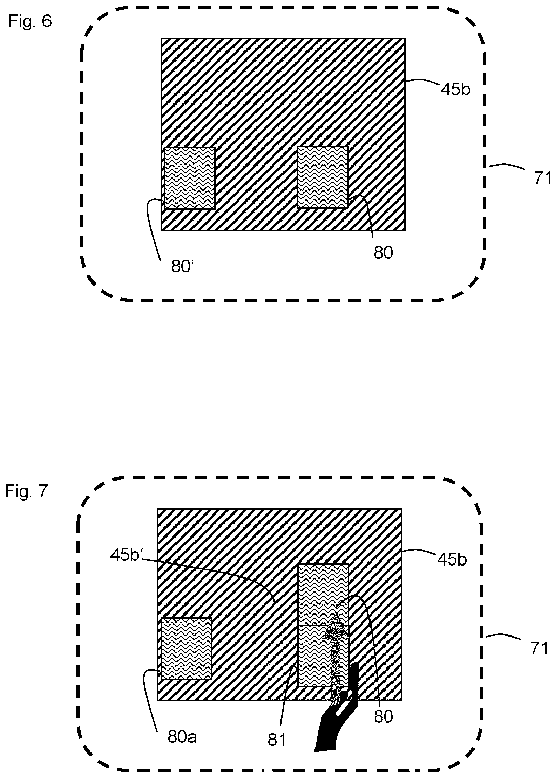

Fig. 6 zeigt einen Lagerbereich, nämlich die Oberseite des Regalblecheinsatzes 45b, aus einer Vogelperspektive bzw. in Aufsicht. Schematisch gezeigt ist zudem der Bilderfassungsausschnitt 71, den die Kamera 70 erfasst. Der Bilderfassungsausschnitt 71 ist der Bereich, der von der Kamera 70 in einem bzw. in jedem Bild erfasst wird, denn die Kamera 70 ändert ihren Blickwinkel und ihre Position nicht. Somit zeigt jedes Standbild, das Startbild, die aus den Videodaten entnehmbaren Einzelbilder, und das Endbild diesen Ausschnitt 71. In den Figuren stellt die untere Kante des Ausschnitts 71 den nahe der Inkubatorkammeröffnung 4 gelegenen Bereich dar.

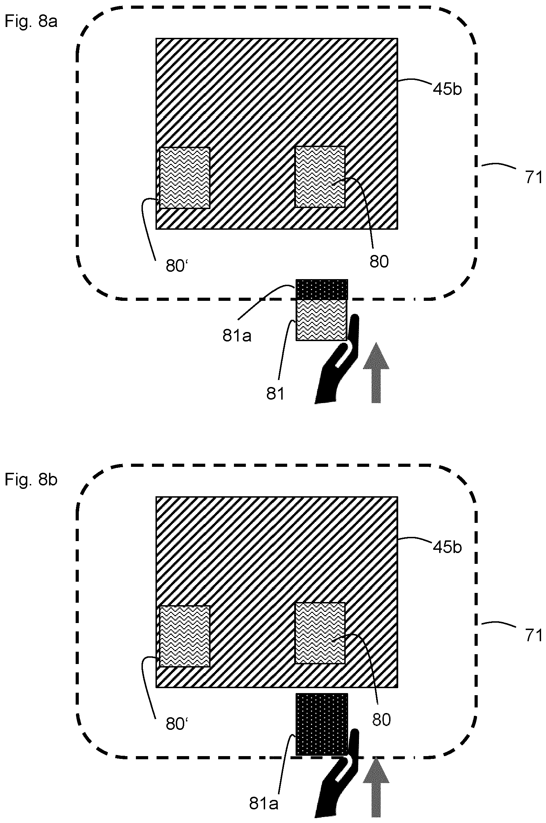

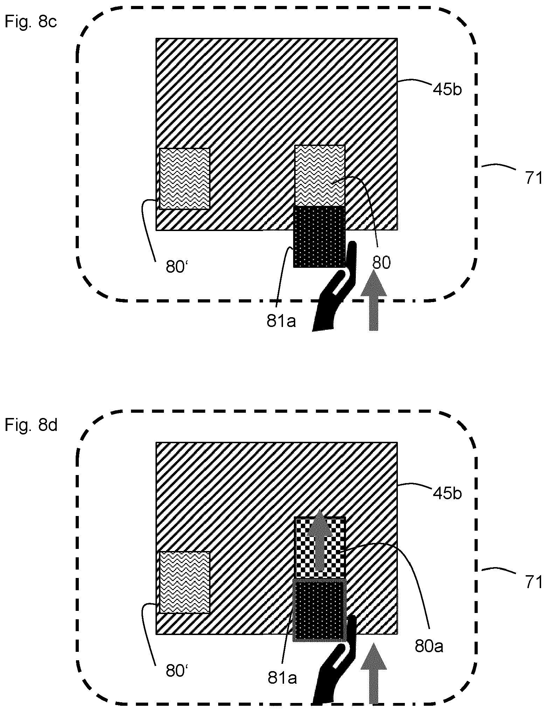

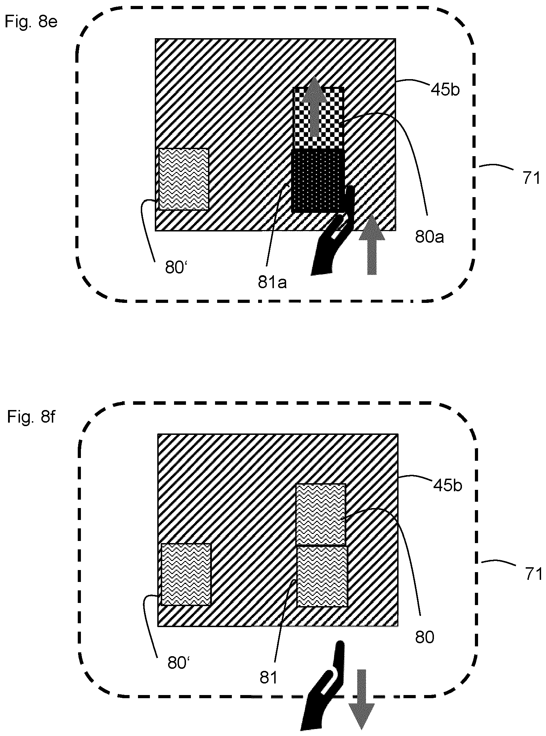

In Fig. 7 ist gezeigt, wie ein Benutzer (gezeigt: dessen Hand) einen Zellkulturbehälter 81 in den Inkubator einstellt, genauer gesagt auf dem Lagerbereich 45b' nach vorne bzw. ins Innere des Inkubators verschiebt. Dabei verschiebt er auch das Bestandsobjekt 80, das äußerlich nicht vom Objekt 81 unterscheidbar ist. Gelangt das neue Objekt 81 in seiner Endposition genau an die bisherige Standposition des Bestandsobjekt 80, so wird aus Standbildern, die vor dem Einstellen und nach dem Einstellen des neuen Objektes 81 erstellt wurden, nicht zwischen dem Objekt 80 und dem Objekt 81 zu unterscheiden sein. Mittels des Objektverfolgungssystems 20, 200 ist dies jedoch möglich, wie anhand der Figuren 8a bis 8e erläutert wird.

Die Datenverarbeitungseinrichtung des Objektverfolgungssystems 20, 200 ist dazu programmiert, mit der Detektion der Türöffnung der Außentüre 41 des Inkubators die Videodaten mittels der Kamera 70 zu erfassen und auszuwerten. Mittels Vergleich von zeitlich aufeinander folgenden Frames der Videodaten kann festgestellt werden, wann ein neues Objekt in den Kameraausschnitt 71 gelangt. Ein Bild, das einen neu im Ausschnitt 71 erscheinenden Umriss 81a enthält, der dem in den Innenraum eingebrachten Objekt 81 zuordenbar ist, wird als Startbild angesehen. Ein solches Bild ergibt sich aus der Situation in Fig. 8a. Ausgehend von diesem Startbild werden dem neu erschienenen Umriss 81a Identifikationsdaten und eine Bounding Box zugeordnet, in der Annahme, dass es sich um ein neu in den Inkubator einzustellendes Objekt 81 handelt. Die Objektverfolgungsalgorithmen des Objektverfolgungssystems 20, 200 verfolgen das durch die Bounding Box definierte Objekt durch die nun folgenden Frames der Videodaten. Aus diesen Frames werden die Positionsänderungen des Objektes 80 durch Auswertung der Videodaten ermittelt. Ignoriert man zunächst den Umstand, dass der Benutzer mittels des neuen Objektes 81 das Bestandsobjekt 80 ausgehend von dessen ursprünglicher Position (dessen früher erfasste Endposition) tiefer in die Inkubatorkammer hineinschiebt, geschieht nun bezüglich der Verfolgung des neuen Objektes 81 folgendes:

In Fig. 8b ist das ganze Objekt als Umriss 81a erkennbar, und auch die Hand des Benutzers selber könnte von der Datenverarbeitungseirichtung 21 zunächst als Teil des Objektes 81 verstanden werden, was aber spätestens beim Entfernen der Hand korrigiert wird. Es werden vom Objektverfolgungssystem die bewegten, und mit dem Umriss 81a verbundenen Bildbestandteile zunächst als Teile des Objektes 81 verstanden. Dieser Umriss 81a wird durch eine Bounding Box definiert, um den Rechenaufwand der Verfolgung zu reduzieren, und wird durch die Frames kontinuierlich verfolgt (Fig. 8a bis Fig. 8e) und Positionsänderungen des Umrisses (der Bounding Box) 81a erfasst, so dass aus jedem Frame die aktuelle Position der Bounding Box 81a entnehmbar ist. Wird keine Bewegung der Bounding Box 81a mehr erfasst, so wird das Bild als "Endbild" der Objektbewegung (so in Fig. 8e) aufgefasst und die Endposition des neuen Objektes 81 wird aus dem Endbild errechnet und die Endposition des Objektes 81 im Lagerbereich in Abhängigkeit von den Identifikationsdaten des mindestens einen Objektes als ID-Positionsdaten in dem Datenspeicher gespeichert.

Als Komplikation wird in dem in den Figuren 8a bis 8e gezeigten Vorgang durch die Bewegung des neuen Objektes 81 das Bestandsobjekt 80 aus seiner bisherigen Position verschoben und das neue Objekt nimmt sogar exakt die bisherige Position des Bestandsobjekts 80 ein. Das Objektverfolgungssystem 20, 200 ermöglicht aber auch in dieser Situation, dass die Objekte 80 und 81 eindeutig unterscheidbar bleiben. Das Objektverfolgungssystem 20, 200 ist dazu eingerichtet und programmiert, eine Vielzahl von Objekten und deren Positionsänderungen, und somit deren Endpositionen nach einer Bewegung gleichzeitig zu verfolgen:
Die Datenverarbeitungseinrichtung 21 ist dazu programmiert,
- dem in den Innenraum eingebrachten mindestens einen Objekt 80 (dem Bestandsobjekt) Identifikationsdaten zuzuordnen - dieser Schritt erfolgte bereits, als das Bestandsobjekt 80 zu einem früheren Zeitpunkt als "neues Objekt 80" in die Inkubatorkammer eingestellt worden war;
- die Startposition des Objektes 80 (Bestandsobjekt) aus dem Startbild des Lagerbereichs und die Positionsänderungen des Objektes 80 (Bestandsobjekt) durch Auswertung der Videodaten zu ermitteln. Dabei ist das Startbild insbesondere ein Standbild, das vor dem Einstellen des Objekts 81 entstanden war, oder ein aus den Videodaten ermitteltes Bild;
- durch Ermittlung der Positionsänderungen des Objektes 80 zwischen dessen Startposition und Endposition das Objekt 80 (Bestandsobjekt) zu verfolgen;
- im Endbild die (neue) Endposition des Objektes 80 im Lagerbereich zu ermitteln; und
- die Endposition des Objektes 80 (Bestandsobjekt) im Lagerbereich in Abhängigkeit von den Identifikationsdaten des Objektes 80 als ID-Positionsdaten in dem Datenspeicher 22 zu speichern.

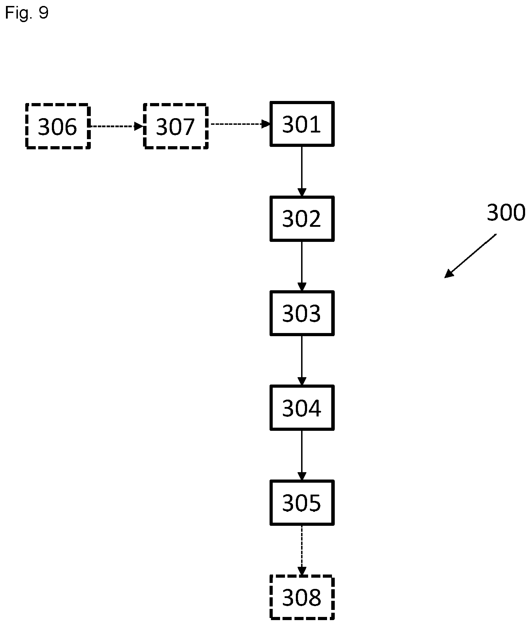

Fig. 9 zeigt den Ablauf des erfindungsgemäßen Verfahrens, das auch schon in der obigen Beschreibung der Figuren 8a bis 8f indirekt genannt war.

Das Verfahren 300 zur Verfolgung der Objektpositionierungen in einem Inkubator 1, welcher der Inkubation von lebenden Zellkulturen in einer Inkubatorkammer 2, 5 des Inkubators dient, aufweisend die computergesteuerten Schritte:
· Überwachen der Inkubatorkammer 2, 5 mittels mindestens einer Kamera des Inkubators, die zur Aufnahme mindestens eines Lagerbereichs 45b' im Innenraums der Inkubatorkammer angeordnet ist, in den das mindestens eine Objekt 80; 81 eingebracht wird; (301)
· Zuordnen von Identifikationsdaten zu dem mindestens einen Objekt 80; 81, das in einem mittels der mindestens einen Kamera aufgenommenen Startbild des Lagerbereichs beim oder nach dem Einbringen in den Innenraum erfasst wird; (302) Erfassen von Positionsänderungen des mindestens einen Objektes 80; 81 zwischen dessen Startposition und Endposition durch Auswertung von mittels der Kamera 70 gewonnenen Videodaten; insbesondere Erfassen von zeitgleichen Positionsänderungen mehrerer Objekte bzw. Bestandsobjekte (303)
· Ermitteln der Endposition des mindestens einen Objektes 80; 81 in einem Endbild des Lagerbereichs; (304)
· Speichern der Endposition des mindestens einen Objektes 80; 81 im Lagerbereich in Abhängigkeit von den Identifikationsdaten des mindestens einen Objektes als ID-Positionsdaten, in dem Datenspeicher. (305)

Vorzugsweise beinhaltet das Verfahren 300 auch die Schritte:
· insbesondere vor den Schritten 301 bis 305, 308: Einlesen von einen den Benutzer des Inkubators 1, der das mindestens eine Objekt 80; 81 in die Inkubatorkammer einbringt, identifizierenden Benutzeridentifikationsdaten mittels einer Benutzeridentifikationseinrichtung (306) und Speichern der Benutzeridentifikationsdaten in einem Datenspeicher des Inkubators. (307)

Vorzugsweise beinhaltet das Verfahren 300 auch den Schritt:
Speichern der ID-Positionsdaten in Abhängigkeit von den Benutzeridentifikationsdaten als benutzerbezogene ID-Positionsdaten. (308)

## Patentansprüche

1. Inkubator zur Inkubation lebender Zellkulturen, aufweisend
eine Inkubatorkammer zur Aufnahme von Objekten, insbesondere Zellkulturbehältern, die eine verschließbare Kammeröffnung zur Auf- und Entnahme der Objekte und mindestens einen Lagerbereich zur Lagerung der Objekte aufweist,
ein bildbasiertes Objektverfolgungssystem zur Verfolgung von Positionsänderungen mindestens eines in die Inkubatorkammer eingebrachten Objektes ausgehend von dessen Startposition in einem Startbild eines Lagerbereichs bis zu dessen Endposition in einem Endbild des Lagerbereichs mittels Videodaten, wobei das Objektverfolgungssystem
mindestens eine Datenverarbeitungseinrichtung, einen Datenspeicher und mindestens eine Kamera aufweist, die dazu eingerichtet ist, einen Innenraum der Inkubatorkammer zu überwachen sowie das Startbild, das Endbild und die Videodaten bereitzustellen,
wobei die Datenverarbeitungseinrichtung dazu programmiert ist,
• dem in den Innenraum eingebrachten mindestens einen Objekt Identifikationsdaten zuzuordnen,
• die Startposition des mindestens einen Objektes aus dem Startbild des Lagerbereichs zu ermitteln;
• die Positionsänderungen des mindestens einen Objektes zwischen der Startposition und einer Endposition durch Auswertung der Videodaten zu ermitteln,
• die Endposition des mindestens einen Objektes im Endbild des Lagerbereichs zu ermitteln, und
• die Endposition des mindestens einen Objektes im Lagerbereich in Abhängigkeit von den Identifikationsdaten des mindestens einen Objektes als ID-Positionsdaten in dem Datenspeicher zu speichern.

2. Inkubator gemäß Anspruch 1, der eine Benutzeridentifikationseinrichtung aufweist, mittels der ein den Inkubator benutzender Benutzer in Form von Benutzeridentifikationsdaten identifizierbar ist.

3. Inkubator gemäß Anspruch 2, wobei die Datenverarbeitungseinrichtung dazu programmiert ist,
• einen den Inkubator benutzenden Benutzer mittels der Benutzeridentifikationseinrichtung zu identifizieren und ihm Benutzeridentifikationsdaten zuzuordnen und
• ID-Positionsdaten in Abhängigkeit von den Benutzeridentifikationsdaten als benutzerbezogene ID-Positionsdaten in dem Datenspeicher zu speichern.

4. Inkubator gemäß Anspruch 2 oder 3, wobei die Benutzeridentifikationseinrichtung eine Außenkamera aufweist, und die Benutzeridentifikationseinrichtung dazu eingerichtet ist, mittels der Außenkamera eine Gesichtserkennung durchzuführen, mittels der der Benutzer identifiziert wird.

5. Inkubator gemäß Anspruch 2, 3 oder 4, wobei die Benutzeridentifikationseinrichtung eine Benutzerschnittstelleneinrichtung aufweist, mittels der Benutzeridentitätsdaten einlesbar, insbesondere auswählbar sind, oder ein Lesegerät zum Einlesen eines den Benutzer identifizierenden Codes aufweist, wobei das Lesegerät insbesondere ein RFID-Lesegerät, ein Barcode-Lesegerät, oder ein QR-Code-Lesegerät ist.

6. Inkubator gemäß einem der vorhergehenden Ansprüche, der aufweist:
• eine Inkubatortüre zum Verschließen der Kammeröffnung und
• einen Türsensor zum Erfassen des Öffnens bzw. des Schließens der Inkubatortüre,
wobei die Datenverarbeitungseinrichtung dazu programmiert ist,
• die Überwachung des Innenraums des Inkubators, insbesondere die Generierung der Videodaten oder von Standbilddaten, in Abhängigkeit von der Detektion einer Türöffnung des Inkubators zu starten,
• insbesondere in Abhängigkeit von der Detektion einer Türöffnung durch einen Benutzer, diesen Benutzer mittels einer Benutzeridentifikationseinrichtung zu identifizieren,
• insbesondere. die Überwachung des Innenraums des Inkubators, insbesondere die Generierung der Videodaten, in Abhängigkeit von der Detektion einer Türschließung des Inkubators zu beenden,
• insbesondere mittels der Informationen aus der Benutzeridentifikationseinrichtung und der Objektverfolgungseinrichtung festzustellen, durch welchen Benutzer welches Objekt im Innenraum bewegt wurde, und die Benutzeridentifikationsdaten dieses Benutzers gemeinsam mit den Objektsidentifikationsdaten dieses Objektes in dem Datenspeicher zu speichern.

7. Inkubator gemäß einem der vorhergehenden Ansprüche, wobei die Datenverarbeitungseinrichtung dazu programmiert ist, aus dem Startbild, den Videodaten und/oder dem Endbild die Bewegungshistorie, insbesondere den Bewegungspfad, des mindestens einen Objektes innerhalb der Inkubatorkammer zu ermitteln und in Form von Bewegungshistoriedaten in dem Datenspeicher zu speichern.

8. Inkubator gemäß einem der vorhergehenden Ansprüche, der ein Objekterkennungssystem aufweist, eingerichtet dazu
* Objektklassenmerkmale des mindestens einen Objektes aus dem Startbild, den Videodaten und/oder dem Endbild zu entnehmen,
* die Objektklassenmerkmale mit einer Objektklassendatenbank abzugleichen und die Objektklasse des mindestens einen Objektes zu erkennen,
* den ID-Positionsdaten des mindestens einen Objektes die erkannte Objektklasse als Objektklassendaten zuzuordnen und als klassenbezogene ID-Positionsdaten zu speichern; oder eingerichtet dazu
* Objektindividualmerkmale des mindestens einen Objektes aus dem Startbild, den Videodaten und/oder dem Endbild zu entnehmen,
* die Objektindividualmerkmale mit einer Objektindividualdatenbank abzugleichen und
# falls die Objektindividualmerkmale in der Objektindividualdatenbank mit einer Objektindividualkennung verbunden sind: die Objektindividualkennung des mindestens einen Objektes zu identifizieren; oder
# falls die Objektindividualmerkmale in der Objektindividualdatenbank nicht mit einer Objektindividualkennung verbunden sind: dem mindestens einen Objekt eine Objektindividualkennung zuordnen und in der Objektindividualdatenbank zu speichern; und
* den ID-Positionsdaten des mindestens einen Objektes die erkannte Objektindividualkennung zuzuordnen und als individualbezogene ID-Positionsdaten zu speichern.

9. Inkubator gemäß einem der vorhergehenden Ansprüche, der einen Bildschirm aufweist, wobei die Datenverarbeitungseinrichtung dazu programmiert ist, eine graphische Wiedergabe des Innenraums der Inkubatorkammer auf dem Bildschirm anzuzeigen, und insbesondere graphisch anzuzeigen, wo das durch die ID-Positionsdaten identifizierte Objekt positioniert ist, bzw. um graphisch anzuzeigen, wo alle im Innenraum lokalisierten Objekte angeordnet sind.

10. Inkubator gemäß Anspruch 9, wobei die Datenverarbeitungseinrichtung dazu programmiert ist, ausgehend von vorgegebenen Benutzeridentifikationsdaten festzustellen, wo die diesen vorgegebenen Benutzeridentifikationsdaten mittels der benutzerbezogenen ID-Positionsdaten zugeordneten Objekte positioniert sind und, insbesondere, diese Objekte auf dem Bildschirm graphisch zu markieren.

11. Inkubator gemäß einem der Ansprüche 9 oder 10, wobei die Datenverarbeitungseinrichtung dazu programmiert ist, auf dem Bildschirm freien Lagerplatz anzuzeigen, und/oder aus den Positionsdaten abgeleitete Informationen, insbesondere die Identität des Benutzers, der die Positionsänderung herbeigeführt hat, und / oder statistische Informationen, insbesondere die Häufigkeit und den Zeitpunkt der Positionsänderung eines Objektes und / oder den prozentual verfügbaren freien Lagerplatz.

12. System zur Inkubation lebender Zellkulturen, aufweisend
einen Inkubator zur Inkubation lebender Zellkulturen, der aufweist: eine Inkubatorkammer zur Aufnahme von Objekten, insbesondere Zellkulturbehältern, in mindestens einem Lagerbereich der Inkubatorkammer, die eine verschließbare Kammeröffnung zur Auf- und Entnahme der Objekte aufweist, eine Datenverarbeitungseinrichtung und einen Datenspeicher,
ein zum Nachrüsten des Inkubators eingerichtetes bildverarbeitendes Objektverfolgungssystem zur Verfolgung von Positionsänderungen mindestens eines in die Inkubatorkammer eingebrachten Objektes ausgehend von dessen Startposition in einem Startbild eines Lagerbereichs bis zu dessen Endposition in einem Endbild des Lagerbereichs mittels Videodaten, wobei das Objektverfolgungssystem
mindestens eine Datenverarbeitungseinrichtung, einen Datenspeicher und mindestens eine Kamera aufweist, die dazu eingerichtet ist, einen Innenraum der Inkubatorkammer zu überwachen sowie das Startbild, das Endbild und die Videodaten bereitzustellen,
wobei die Datenverarbeitungseinrichtung dazu programmiert ist,
• dem in den Innenraum eingebrachten mindestens einen Objekt Identifikationsdaten zuzuordnen;
• die Startposition des mindestens einen Objektes aus dem Startbild des Lagerbereichs zu ermitteln,
• die Positionsänderungen des mindestens einen Objektes zwischen der Startposition und einer Endposition durch Auswertung der Videodaten zu ermitteln,
• die Endposition des mindestens einen Objektes im Endbild des Lagerbereichs zu ermitteln, und
• die Endposition des mindestens einen Objektes im Lagerbereich in Abhängigkeit von den Identifikationsdaten des mindestens einen Objektes als ID-Positionsdaten in dem Datenspeicher zu speichern.

13. System nach Anspruch 12, aufweisend
eine getrennt vom Inkubator verwendbare Benutzeridentifikationseinrichtung und eine Datenaustauscheinrichtung, mittels der die Datenverarbeitungseinrichtung anhand der Benutzeridentifikationseinrichtung Benutzeridentifikationsdaten ermitteln kann.

14. Verfahren zur Verfolgung der Objektpositionierungen in einem Inkubator, welcher der Inkubation von lebenden Zellkulturen in einer Inkubatorkammer des Inkubators dient, aufweisend die computergesteuerten Schritte:
• Überwachen der Inkubatorkammer mittels mindestens einer Kamera des Inkubators, die zur Aufnahme mindestens eines Lagerbereichs im Innenraum der Inkubatorkammer und zur Erstellung des Startbildes, des Endbildes und von Videodaten angeordnet ist, in den das mindestens eine Objekt eingebracht wird;
• Zuordnen von Identifikationsdaten zu dem mindestens einen Objekt, das in einem mittels der mindestens einen Kamera aufgenommenen Startbild des Lagerbereichs beim oder nach dem Einbringen in den Innenraum erfasst wird;
• Erfassen von Positionsänderungen des mindestens einen Objektes zwischen der Startposition und einer Endposition durch Auswertung von mittels der Kamera gewonnenen Videodaten;
• Ermitteln der Endposition des mindestens einen Objektes in einem Endbild des Lagerbereichs;
• Speichern der Endposition des mindestens einen Objektes im Lagerbereich in Abhängigkeit von den Identifikationsdaten des mindestens einen Objektes als ID-Positionsdaten, in dem Datenspeicher.

15. Verfahren nach Anspruch 14, aufweisend den Schritt:
Einlesen von einen den Benutzer des Inkubators, der das mindestens eine Objekt in die Inkubatorkammer einbringt, identifizierenden Benutzeridentifikationsdaten mittels einer Benutzeridentifikationseinrichtung und Speichern der Benutzeridentifikationsdaten in einem Datenspeicher des Inkubators.

16. Verfahren nach Anspruch 14, aufweisend den Schritt:
Speichern der ID-Positionsdaten in Abhängigkeit von den Benutzeridentifikationsdaten als benutzerbezogene ID-Positionsdaten.

17. Verfahren gemäß der Ansprüche 14 bis 16, aufweisend den Schritt: Visualisierung der gespeicherten ID-Positionsdaten und/oder der benutzerbezogenen ID-Positionsdaten auf dem Bildschirm, insbesondere durch eine graphische Wiedergabe des Innenraums der Inkubatorkammer auf dem Bildschirm, und insbesondere das graphische Anzeigen der Information, wo das durch die ID-Positionsdaten identifizierte Objekt positioniert ist, bzw., wo alle im Innenraum lokalisierten Objekte angeordnet sind, oder Anzeigen des freien Lagerplatzes im Innenraum des Inkubators auf dem Bildschirm.
